# EUROPÄISCHE PATENTSCHRIFT

(11) **EP 1 741 666 B1**
(45) Veröffentlichungstag und Bekanntmachung des Hinweises auf die Patenterteilung: **11.10.2017**
(21) Anmeldenummer: 06012711.5
(22) Anmeldetag: 21.06.2006
(51) Int. Cl.: B67C 7/00

(54) **Verfahren und Maschine zum Verschliessen von Flaschen mit sterilen Kappen**
Method and device for closing bottles with sterile caps
Méthode et dispositif pour fermer des bouteilles avec des capsules stériles

(30) Priorität: 08.07.2005 DE 10532322
(43) Veröffentlichungstag der Anmeldung: 10.01.2007
(73) Patentinhaber: KHS Corpoplast GmbH, 22145 Hamburg (DE)
(72) Erfinder: Kemper, Berthold, 48683 Ahaus (DE); Niehr, Thomas, 41812 Erkelenz (DE)
(74) Vertreter: Hausfeld, Norbert

(56) Entgegenhaltungen:
- WO-A1-2004/033300
- WO-A2-2005/108278
- DE-A1- 19 851 654
- DE-U1- 9 310 736
- GB-A- 2 007 630
- US-A- 4 304 611

## Beschreibung

Die Erfindung betrifft ein Verfahren zum Verschließen von Flaschen mit sterilen Kappen gemäß dem Oberbegriff des Anspruchs 1 sowie eine Maschine gemäß dem Oberbegriff des Anspruchs 12.

Bei einem aus der gattungsgemäßen EP 0 993 418 B1 bekannten Verfahren zum Verschließen von Flaschen werden die Kappen entlang einer Transportbahn in einen Sterilbereich zugeführt und danach erfolgt in einer vollständig in einer Sterilkammer angeordneten Übergabevorrichtung eine Sterilisation. Die mittels eines Rundläufers vereinzelt aufgenommenen Kappen werden mit nach unten weisendem Innenraum innerhalb der Sterilkammer geschwenkt und so auf die Flaschen aufgesetzt, dass diese danach mittels eines Schließorgans verschlossen werden kann.

Im Bereich der Lebensmittelverpackung sind weitere Verfahren bekannt, bei denen zur Verschraubung vorgesehene Kappen nach der Sterilisation von einem entsprechenden Verschraubungswerkzeug erfasst, in den Bereich einer Flaschenzuführung verlagert und auf die Flasche geschraubt werden.

Zur Sterilisation von Kappen, Flaschen, Behältern o. dgl. Transportmitteln sind mit Wasserstoffperoxid arbeitende Verfahren bekannt, um die extern sterilisierten Innenräume der Behälter anschließend unter aseptischen Bedingungen mit Lebensmitteln o. dgl. zu füllen. Gefüllte Flaschen werden anschließend mit einem Aluminiumsiegel steril verschlossen und außerhalb einer Füllmaschine wird in einem zusätzlichen Verfahrensschritt die Kappen aufgebracht. Derartige Abfüll- und Schließmaschinen werden konstruktiv in Rundläufer und Linearläufer unterteilt, wobei ein Versiegeln oder ein Verschrauben vorgesehen sein kann. Bei einer Verschraubung in bekannten Maschinen werden die Kappen mittels Peressigsäure in einem Sterilisationsbad entkeimt, wobei jedoch die Gefahr besteht, dass in den Kappen enthaltene Gleitmittel ausgewaschen werden und dadurch ein späteres Öffnen der Flasche erschwert ist. Andere Verfahren verwenden Wasserstoffperoxid-Aerosol zur Sterilisation (EP 0 993 418 B1), wobei die Kappen einer mit Wasserstoffperoxid gesättigten Gasatmosphäre ausgesetzt werden kann. Diese Verfahren laufen in einem kontinuierlichen Verfahren mit Rundläufern ab.

Die US 4,304,611 A beschreibt Verfahren und Vorrichtungen zum Reinigen von Kappen. Dabei werden die zu reinigenden Kappen mit Wasser und Luft beaufschlagt, z.B. um Staubreste zu entfernen. Innerhalb des Gehäuses der gezeigten Vorrichtungen wird ein Unterdruck erzeugt, um den Abfluss des Reinigungswassers durch einen Abflusskanal zu fördern und um das Ausdringen von Spritzwasser aus einem Ladekanal zu verhindern. Weder die Kappen noch die Flaschen werden in einen sterilen Zustand versetzt oder unter Sterilbedingungen verarbeitet.

Die nachveröffentliche WO 2005/108278 A2 betrifft Verfahren und Vorrichtungen zum sterilen Füllen und Verschließen von Flaschen.

Die Erfindung befasst sich mit dem Problem, ein Verfahren und eine Maschine zum Verschließen von Flaschen mit sterilen Kappen zu schaffen, wobei die Kappen in einem linearen Verfahrensablauf taktweise sowohl bei vertikalen Hubbewegungen als auch bei vertikalen Schraubbewegungen unter sterilen Bedingungen einfacher und mit höherer Durchsatzleistung handhabbar sind und dabei ein besserer Schutz gegen Rekontaminationen im Bereich der Kappen und Flaschen möglich ist.

Die Erfindung löst dieses Problem mit einem Verfahren mit den Merkmalen des Anspruchs 1 und einer Maschine mit den Merkmalen des Anspruchs 12. Weitere Ausgestaltungen ergeben sich aus den Ansprüchen 2 bis 11 bzw. 13 bis 40.

Bei dem erfindungsgemäßen Verfahren zum Verschließen von Flaschen werden die Kappen bereits außerhalb einer Sterilisationsstrecke so aufgereiht, dass bei einer nachfolgend über eine vertikale Führungsbahn erfolgenden Bewegung der Kappen eine einfache Beschickung einer Sterilisationsstrecke erreicht wird. In deren Bereich ist eine mit geringem Bauraum ausführbare Sterilisationsvorrichtung vorgesehen, die bei insgesamt einfachem Aufbau einen sicheren Schutz vor Rekontamination gewährleistet. Die im Bereich der nachrutschenden Kappen vorgesehenen Ein- und

Auslaßöffnungen zur kurz bemessenen Sterilisationsstrecke weisen vorteilhaft geringe Abmessungen auf. Dabei wird mittels eines entsprechenden Belüftungssystems im Innenraum dieser Sterilisationsstrecke erzeugte Sterilluft mit Überdruck so geleitet, daß zusätzlich zur effizienten Spülreinigung der Kappen mit dem abströmenden Gemisch ein permanenter Reinigungseffekt sowohl an den nachrückenden Kappen als auch im Bereich der Flaschen bewirkt ist.

Der gesamte Verschließprozeß ist darauf abgestellt, daß die aseptischen Kappen durch ein Pic-and-Place-System auf die Flaschen aufgelegt werden, diese danach gegen Rekontamination geschützt sind und die dichtende Verschlußphase mit den eine Rekontaminationsgefährdung bewirkenden Schließbaugruppen einem weiteren Sterilbereich zugeordnet sind.

Ausgehend von diesem Konzept einer Sterilisationsvorrichtung mit vertikaler Transportbahn sind jeweilige an diese anschließende Baugruppen der Maschine so angeordnet, daß mit diesen modularen Teilen ein getakteter Linearläufer gebildet wird. Dieser ist im Bereich einer unmittelbar an die Sterilisationsvorrichtung anschließenden Aufsatzvorrichtung auf engstem Raum mit einer horizontalen Flaschenzuführung verbunden. Bereits in diesem mittels Sterilluft rekontaminationsfrei gehaltenen zweiten Sterilbereich kann mittels eines einfachen Aufsetzorgans ein fluiddichter Verschluß der Flaschen hergestellt werden. Dabei ist beispielsweise eine einfache Rastverbindung zwischen der sterilisierten Kappe und der zugeführten Flasche denkbar.

Ebenso ist eine Konstruktionsvariante der Maschine denkbar, bei der die als Kappen-Flaschen-Einheit zusammengeführten Teile in einen nachfolgenden dritten Sterilbereich weitergegeben wird. In zweckmäßiger Ausführung ist dazu das Maschinenkonzept so ausgelegt, daß im Bereich der Aufsetzvorrichtung die Flaschen mit der Kappe nur zu der kontaminationsdichten Einheit verbunden werden und diese nach Weiterförderung zu einer im dritten Sterilbereich befindlichen Schließvorrichtung hin endgültig verschlossen wird, wobei ein an sich bekannter Schraubverschluß, ein. Schweißverschluß o. dgl. vorgesehen sein kann.

Weitere Einzelheiten und Wirkungen ergeben sich aus der nachfolgenden Beschreibung und den Zeichnungen, in denen eine zur Durchführung des Verfahrens vorgesehene Ausführung der erfindungsgemäßen Maschine veranschaulicht ist. In der Zeichnung zeigen:
- Fig. 1: eine Seitenansicht der erfindungsgemäßen Maschine als Teil einer nicht näher dargestellten Füllanlage für Flaschen,
- Fig. 2: eine Vorderansicht der Maschine gemäß Fig. 1,
- Fig. 3: eine Schnittdarstellung der Maschine gemäß einer Linie III-III in Fig. 2,
- Fig. 4: eine geschnittene Vorderansicht der Maschine gemäß einer Linie IV-IV in Fig. 3,
- Fig. 5: eine Schnittdarstellung ähnlich Fig. 4 gemäß einer Linie V-V in Fig. 3,
- Fig. 6: eine vergrößerte Ausschnittsdarstellung der Maschine im Bereich einer die zugeführten Kappen sterilisierenden Vorrichtung,
- Fig. 7: eine vergrößerte Prinzipdarstellung der Sterilisationsvorrichtung gemäß Fig. 6,
- Fig. 8: eine vergrößerte Ausschnittsdarstellung des unteren Endbereichs der Sterilisationsvorrichtung im Übergang zu einem zweiten Sterilbereich,
- Fig. 9: eine Schnittdarstellung der Sterilisationsvorrichtung gemäß einer Linie IX-IX in Fig. 7,
- Fig. 10 bis Fig. 16: jeweilige vergrößerte Ausschnittsdarstellung des zweiten Sterilbereichs mit Übergabephasen der sterilen Kappe zur Aufsetzvorrichtung,
- Fig. 17: eine vergrößerte Ausschnittsdarstellung eines eine Schraubvorrichtung aufweisenden dritten Sterilbereichs, und
- Fig. 18 und Fig. 19: jeweilige Bewegungsphasen der Schraubvorrichtung beim Aufschrauben der Kappe auf die Flasche.

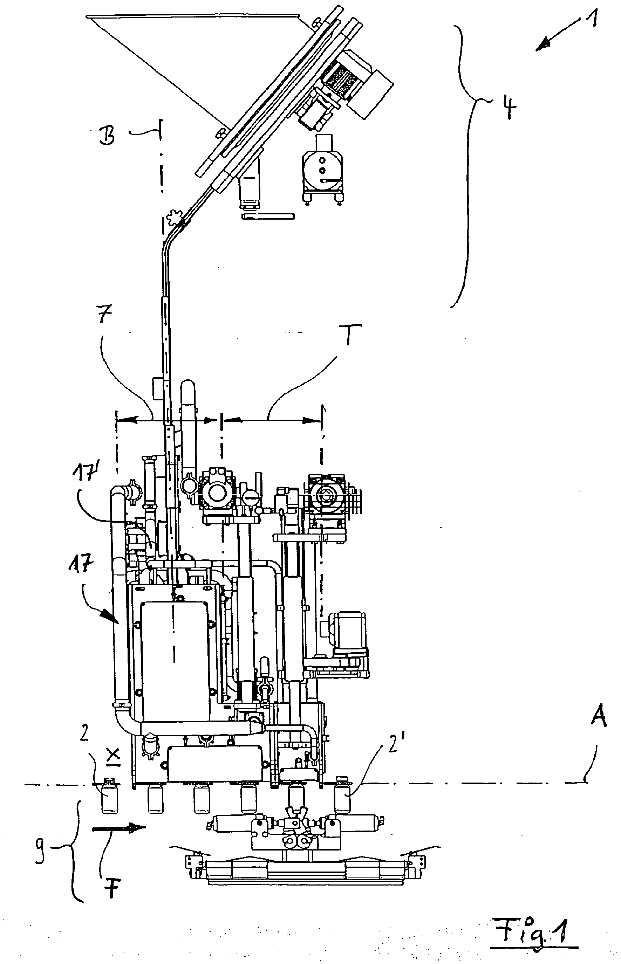

Eine Maschine 1 (Fig. 1) zum Verschließen von Flaschen 2 mit sterilen Kappen 3 nutzt beim Schließverfahren die an sich bekannte Kappenzuführung über eine Transportbahn 5 als Übergabevorrichtung in einen Sterilbereich S, aus dem dann die Flaschen 2 mit nach unten weisendem Innenraum 18 auf die Flaschen 2 aufgesetzt werden können.

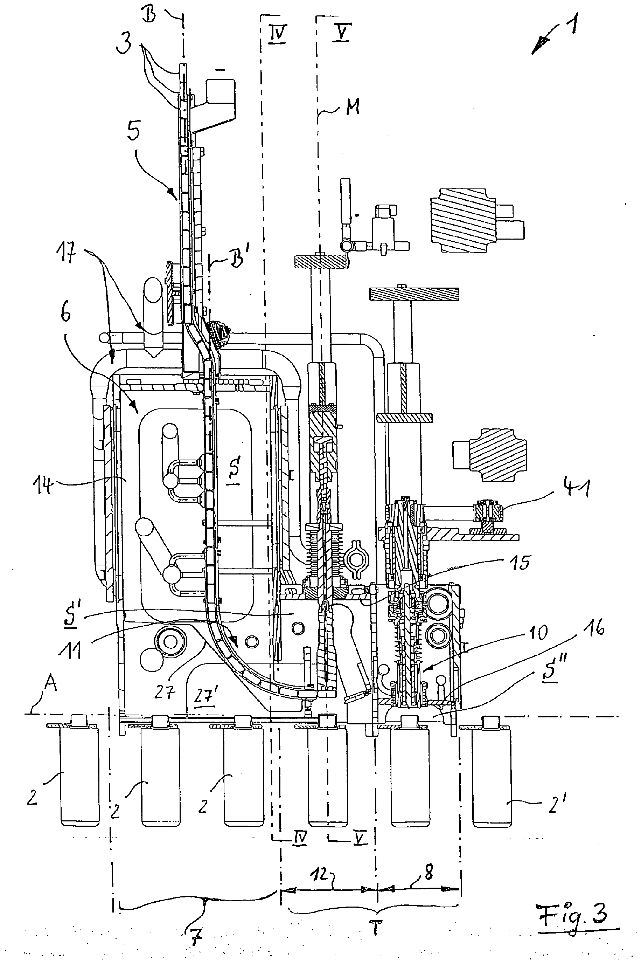

In erfindungsgemäßer Ausführung ist ein derartiger Verschließprozeß (Fig. 3) dadurch verbessert, daß die Kappen 3 in einer unsterilen Umgebung in eine vertikale Reihung verbracht und in dieser Förderlage mit in horizontaler Richtung offenem Innenraum 18 unter Beibehaltung der vertikalen Vorschubrichtung (Ebene B) dem ersten Sterilbereich S zugeführt wird. Nach einer Sterilisierung der Kappen 3 in diesem Bereich S folgt eine unmittelbare Überführung in einen zweiten Sterilbereich S', in dem ein Aufsetzen auf in diesen Sterilbereich S' linear zugeführte Flaschen 2 erfolgt. Danach können die im Sterilbereich S' gebildeten Flaschen-Kappen-Einheiten wahlweise sofort verschlossen oder nach dem Aufsetzen weitergefördert und dann zeitnah verschlossen werden.

Die Flaschen-Kappen-Einheiten können sofort im zweiten Sterilbereich S' mit einer fortgesetzten vertikalen Relativbewegung zwischen Kappe 3 und Flasche 2 zu der dichten Einheit verschlossen werden oder es wird in einem dritten Sterilbereich S" mit den bereits aufgesetzten Kappen der dichte Flaschenverschluß durch eine Schraubbewegung, einen Schweißvorgang o. dgl. realisiert. Damit wird insgesamt ein Linearläufer-System geschaffen, dessen Verschließprozeß eine Aufsetzphase und eine Verschließphase aufweist. Ausgehend von der eine komplett offene Flasche 2 zuführenden Transportphase folgt die Aufsetzphase der Kappe 3 und erst danach eine bewegungsintensive Baugruppen erfordernde Phase des Verschließprozesses. Damit wird eine räumliche Entzerrung, z. B. durch einen zusätzlichen Fördertakt, erreicht und unter optimalen Bedingungen die Sicherheit des aseptischen Verschließprozesses erheblich erhöht, dadurch, daß eine Rekontamination der Kappen 3 bzw. der Flaschen 2 in deren Öffnungsbereich durch die sich bewegenden Baugruppen vermieden ist.

Verfahrensgemäß ist fei wählbar, ob der Flaschenverschluß durch eine einen Schub-Rast-Vorgang nutzende Relativbewegung zwischen Kappe 3 und Flasche 2 erfolgt oder diese durch einen Schraubvorgang, einen Schweißvorgang o. dgl. miteinander verbunden werden.

Die außerhalb des ersten Sterilbereiches S mit horizontal ausgerichtetem Innenraum 18 in jeweilige vertikale Bereiche (Ebene B) von Transportbahnen 5 einsortierten Verschlußkappen 3 werden dabei in einen vertikal zu durchlaufenden ersten Sterilbereich S getaktet weitergeleitet, wonach in einer horizontalen Sprührichtung C eine Sterilisation mit insbesondere Wasserstoffperoxid (H₂O₂) erfolgt. Danach werden die Kappen 3 auf einer eine Einwirkzeit vorgebenden Förderstrecke L zu einem Sprüh- bzw. Spülbereich und einem Trocknungsbereich geleitet und in vorteilhafter Ausführung unmittelbar auf der Transportbahn 5 in eine Stellung mit nach unten weisendem Innenraum 18 umgelenkt. In dieser Stellung gelangen die Kappen 3 in den zweiten Sterilbereich S' und werden hier auf die Flaschen 2 aufgesetzt.

Die Maschine 1 ist im Bereich der Sterilisationsvorrichtung 7 so konzipiert, daß bereits während der vertikalen Zuführphase der Kappen 3 eine Vorentkeimung an deren Außen- und Innenseite erreicht wird, wobei ein aus dem ersten Sterilbereich S abströmendes Luft-Sterilisationsmittel-Gemisch 25 effizient genutzt wird. Durch eine entsprechende Konturführung im Bereich der Transportbahn 5 ist sichergestellt, daß bereits in dieser Zuführphase auch der Innenraum 18 der Kappen 3 besprüht werden kann.

Bei der Sprüh- bzw. Spülbehandlung der Kappen 3 beim Durchlaufen des ersten Sterilbereiches S wird das zumindest phasenweise zugeführte Sterilisationsmittel mit Überdruck in den Innenraum 18 der Kappen 3 eingebracht und nach einer durch die Taktung des Systems sowie den Abstand von Sprüh- und Trocknungsbereich bedingte und weitgehend variabel anpaßbare Einwirkzeit erfolgt eine Trocknung durch Ausblasen des Innenraums 18 der Kappen 3.

Durch eine entsprechende Bemessung der Förderstrecke im Bereich der Transportbahn 5 ist eine Verfahrensführung möglich, bei der im ersten Sterilbereich gleichzeitig mehrere der in der Transportbahn zugeführten Kappen 3 unter Überdruck sterilisiert werden, so daß entsprechend der Taktung im Sprühbereich beispielsweise drei Sprühtakte zu durchlaufen sind und danach ein ebenfalls mehrphasiger Trocknungsbereich durchlaufen wird.

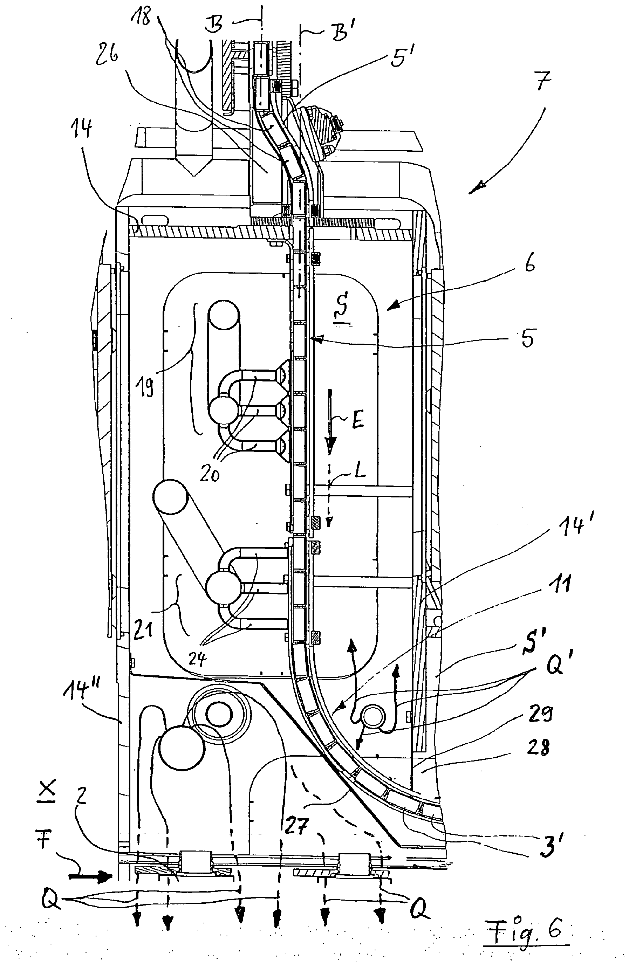

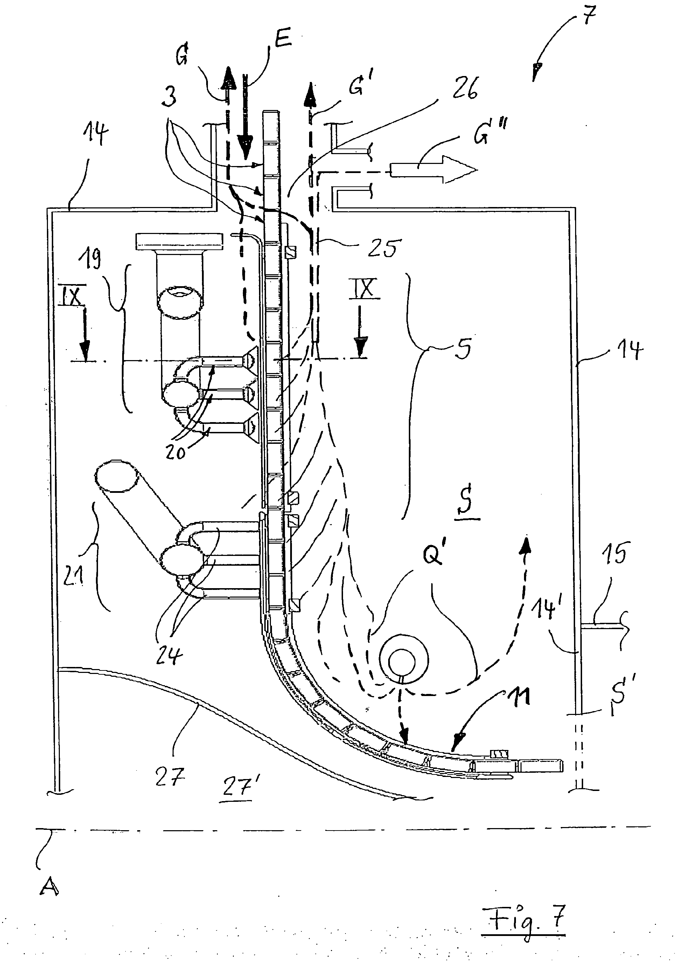

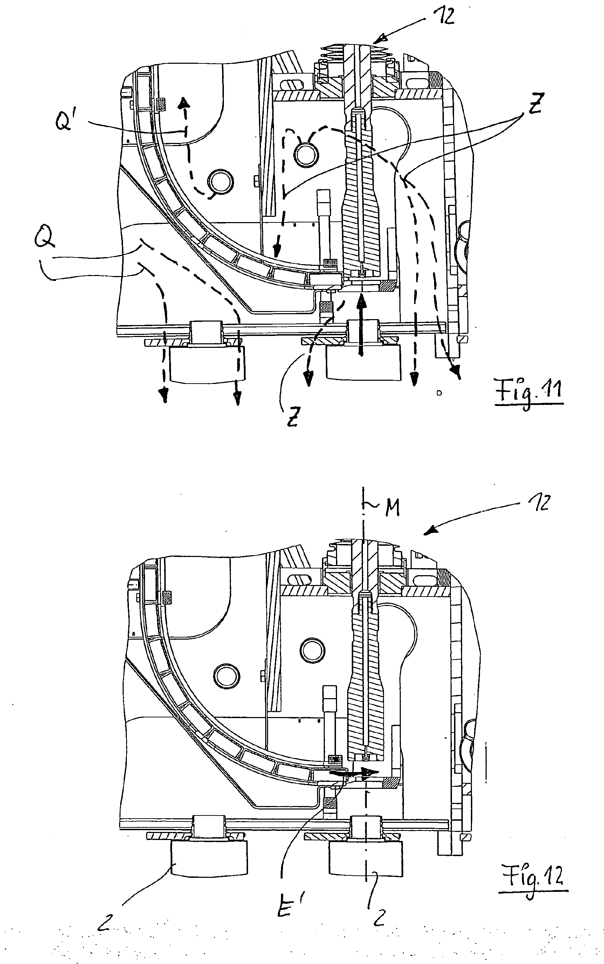

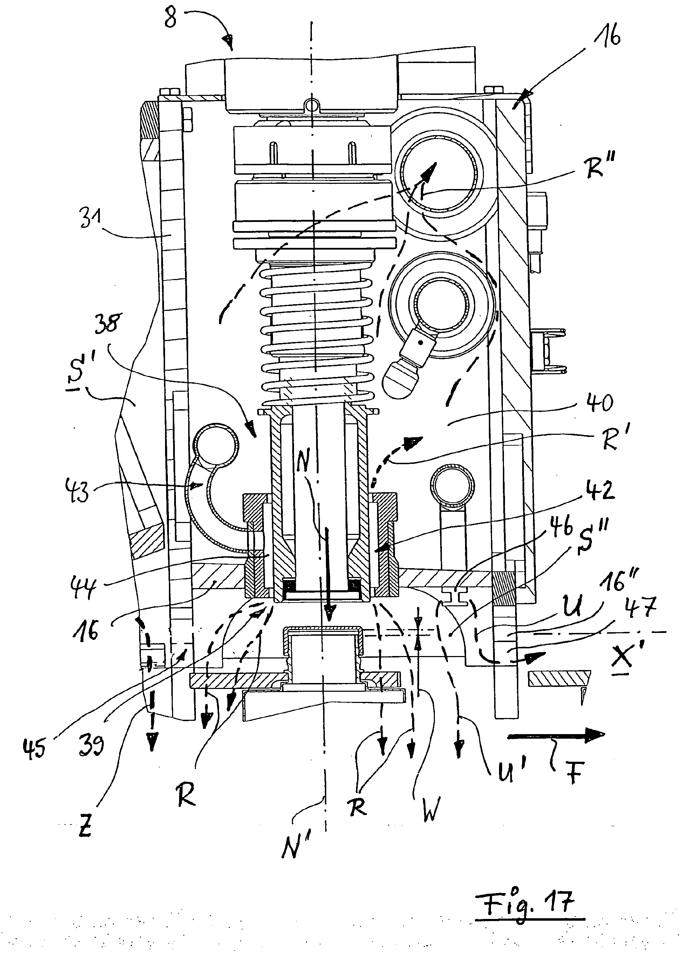

Die insgesamt eine kompakte Reihenanordnung ihrer Baugruppen aufweisende Linearläufer-Maschine 1 erlaubt eine optimale Steuerung der zur Verfahrensführung erforderlichen Sterilluft, wobei durch deren optimale Ausnutzung ein effizienter Rekontaminationsschutz erreicht wird. Dabei ist vorgesehen, daß der vertikale erste Sterilbereich S, der den Aufsetzbereich bildende zweite Sterilbereich S' und der insbesondere zur Verschraubung der Kappen 3 vorgesehene dritte Sterilbereich S" durch jeweilige einzeln erzeugte und sterile Luft und/oder Sterilisationsmittel enthaltende Verdrängungsströmungen vor Rekontaminationen geschützt werden. In Fig. 6 (Strömungen gemäß Pfeil Q und Q'), Fig. 7 (Strömungen gemäß Pfeil G, G', G"), Fig. 11 (Strömungen gemäß Pfeil Z) und Fig. 17 (Strömungen gemäß Pfeil R, R' und U, U') sind diese Bedingungen beispielhaft verdeutlicht. Wesentlich ist dabei auch, daß in einer Zone zwischen erstem und zweitem Sterilbereich S, S' jeweilige eine Rekontamination sowohl der Kappen 3 als auch der bereits gefüllt zugeförderten Flaschen 2 verhindernde Verdrängungsströmungen, beispielsweise mittels steriler Luft, eingeleitet werden (Strömungen Q und Z). Damit ist bereits beim Aufsetzen der Kappen 3 ein aseptischer Verschluß der Flaschen 2 garantiert.

Bei der Verfahrensführung ist vorgesehen, daß zumindest im Aufsetzbereich der Kappen 3 auf die Flaschen 2 eine Sterilluftzuführung erfolgt und ausgehend von dieser eine Ableitung (Pfeil Z) über die auf der horizontalen Zuführebene A einlaufenden Flaschen 2 erfolgt. Denkbar ist auch, daß eine Ableitung (Pfeil Q') in den höher gelegenen ersten Sterilbereich S erfolgt. In vorteilhafter Ausführung der Strömungsführungen ist vorgesehen, daß diese eine laminare Strömung erzeugen und im Bereich zwischen den Schließphasen eine vertikale Ableitung ohne Querströmungen oder Turbulenzen wirksam ist.

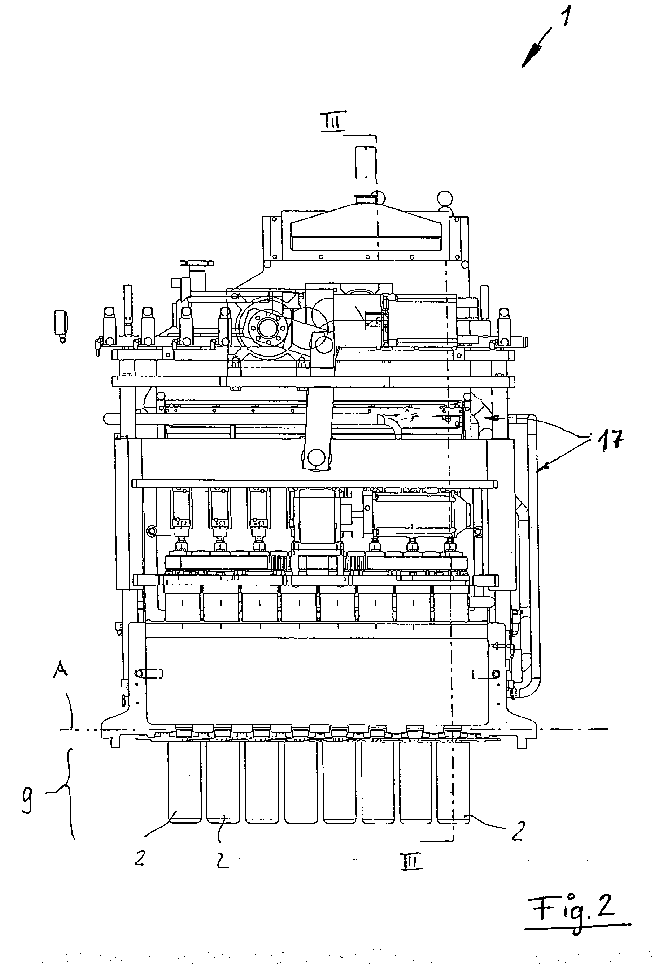

Es versteht sich, daß das im wesentlichen anhand einer Zuführbahn B bzw. Zuführebene A erläuterte Verfahren und die damit konzipierte Maschine 1 eine mehrbahnige Anordnung der Vorrichtungsbaugruppen aufweist. Insbesondere ist vorgesehen, daß die Kunststoff-Flaschen 2 und die Kappen 3 durch eine 8-bahnige Zuführung (Fig. 2) jeweils getaktet in dem zweiten Sterilbereich S" zusammengeführt und aus diesem als die Flaschen-Kappen-Einheit zum endgültigen Verschluß weitergeleitet werden.

In Fig. 1 ist eine insgesamt mit 1 bezeichnete Maschine als Teil einer nicht näher dargestellten linearen Abfüllanlage zum sterilen Verschließen von Flaschen 2 mit Kappen 3 dargestellt. Deutlich wird dabei, daß die Kappen 3 ausgehend von einer an sich bekannten Vereinzelungsvorrichtung 4 auf einer allgemein mit 5 bezeichneten Transportbahn in einer eine Sterilkammer 6 aufweisenden Sterilisationsvorrichtung 7 sterilisiert, unter sterilen Bedingungen auf in einer horizontalen Zuführebene A zugeführte Flaschen 2 aufgesetzt und diese danach mittels einer Schließvorrichtung 8 verschlossen werden.

Bei dem erfindungsgemäßen Konzept der Maschine 1 ist vorgesehen, daß die Sterilisationsvorrichtung 7 durch eine oberseitige Vereinzelungsvorrichtung 4 und eine eine vertikale Ebene B definierende Transportbahn 5 mit der Sterilisationsvorrichtung 7 verbunden ist. Diese Sterilisationsvorrichtung 7 wirkt mit einer ausgangsseitig die Kappen 3 übernehmenden sowie mit einer horizontalen Flaschenzuführung 9 verbundenen Aufsetz- und/oder Schließeinheit T zusammen. Diese VorrichtungsBaugruppen 4 und 7 sowie T mit einer zumindest ein Schließorgan 10 aufweisenden Schließvorrichtung 8 bilden insgesamt ein getaktetes Aggregat nach Art eines Linearläufers (Fig. 3).

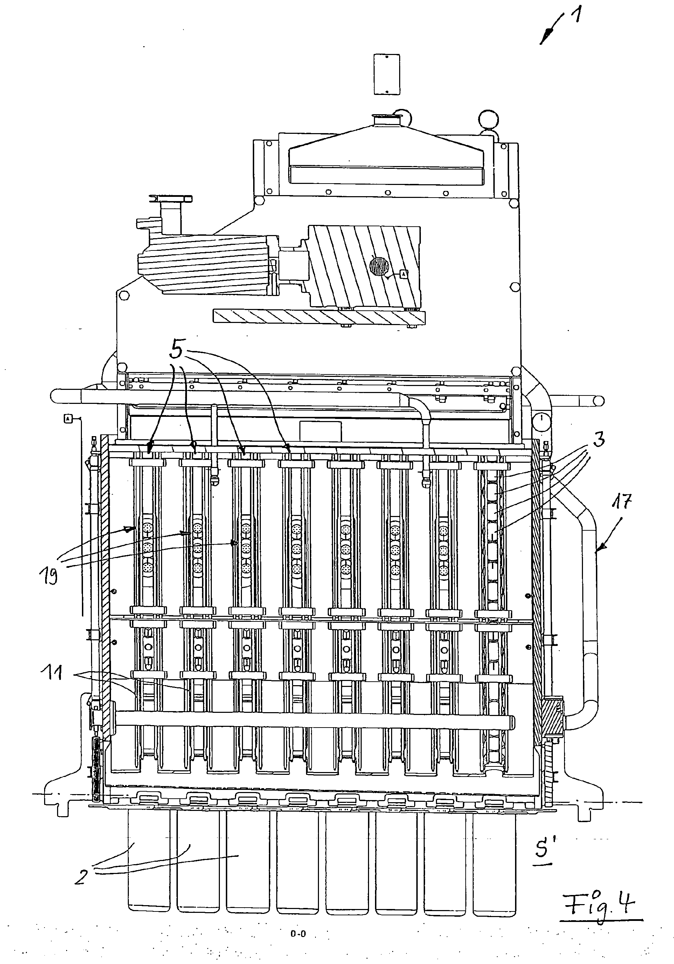

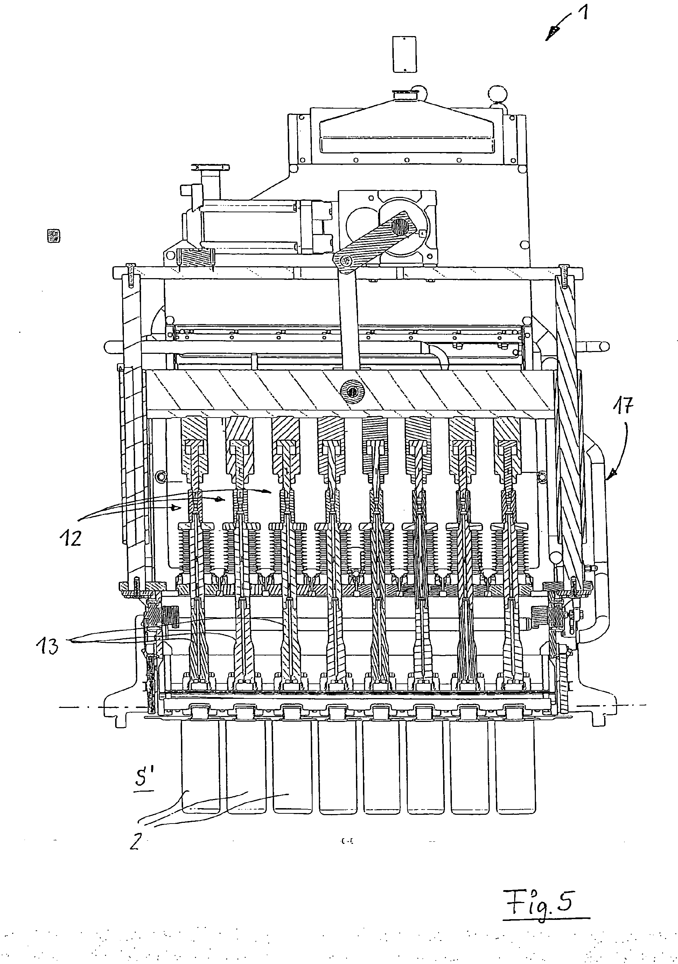

In den Darstellungen gemäß Fig. 2 bis 5 zeigen unterschiedliche Ansichten den prinzipiellen Aufbau dieser Linearläufer-Maschine 1, die insbesondere für eine H₂O₂-Sterilisation in mehrbahniger, vorzugsweise 8-bahniger, Ausführung parallele Verschließprozesse realisiert (Fig. 2, Fig. 4, Fig. 5). Mit einem in getrennten Sterilgehäusen ablaufenden Zwei-Schritt-Verschließprozeß wird in einer vorteilhaft kompakt aufgebauten Einheit mit vermindertem technischem Aufwand eine verbesserte Hygiene erreicht.

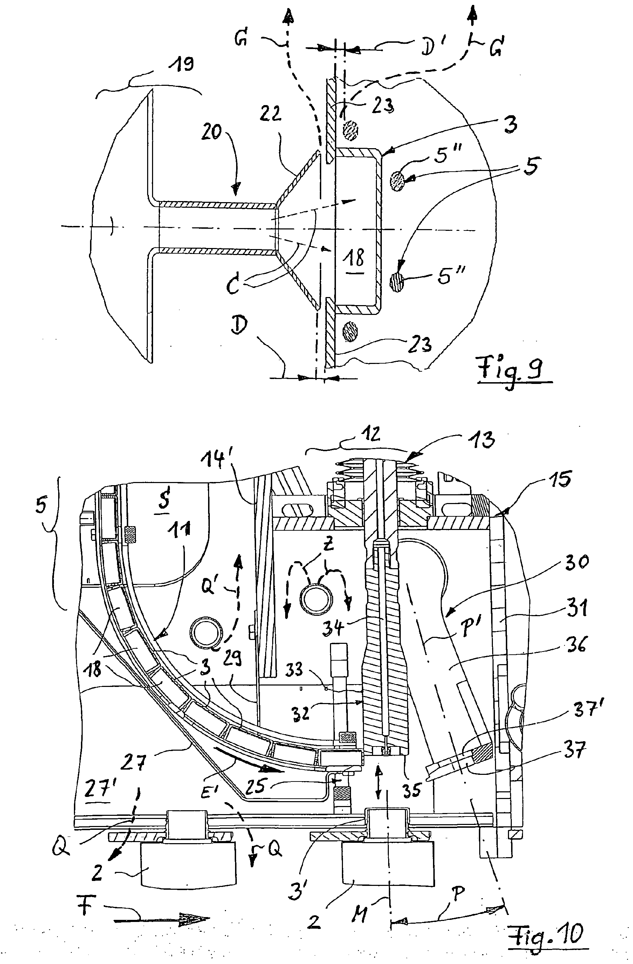

Die Schnittdarstellung gemäß Fig. 3 zeigt in Zusammenschau mit Fig. 6 und Fig. 7 jeweilige Verlagerungsphasen beim Zuführen der Kappen 3, wobei diese auf einer Transportbahn 5 vertikal durch eine insgesamt als Teilbereich 7 bezeichnete Sterilisationsvorrichtung mit Sterilkammer 6 (Fig. 3) geleitet werden, in der ein erster Sterilbereich S gebildet ist. Am unteren Ende der Transportbahn 5 ist ein Umlenkabschnitt 11 der Bahn 5 vorgesehen, der im Nahbereich einer im zweiten Sterilbereich S' angeordneten Aufsetzvorrichtung 12 ausmündet. Diese Aufsetzvorrichtung 12 weist dabei ein Aufsetzorgan 13 auf (Fig. 10 bis 16), das in der Linearläufer-Maschine 1 erfindungsgemäßer Ausführung sowohl zum Aufsetzen als auch zum Schließen ausgebildet sein kann. An Stelle des die Kappen 3 unter Schwerkraftwirkung weiterleitenden Umlenkabschnittes 11 ist auch denkbar, einen die Kappen 3 in den Sterilbereich S' übergebenden Schwenkmechanismus o. dgl. Förderelemente vorzusehen (nicht dargestellt).

Unmittelbar nachgeordnet dem zweiten Sterilbereich S' ist ein die Schließvorrichtung 8 mit dem Schließorgan 10 aufweisender dritter Sterilbereich S" vorgesehen, wobei die Sterilisationsvorrichtung 7, die Aufsetzvorrichtung 12 und die Schließvorrichtung 8 in jeweiligen in der Schnittdarstellung gemäß Fig. 3 verdeutlichten Sterilgehäusen 14, 15, 16 angeordnet sind. Diese Baugruppen sind vorteilhaft oberhalb eines die Flaschen 2 horizontal zuführenden Flaschenförderteils der Flaschenzuführung 9 so positioniert, daß eine unmittelbare Reihenanordnung der Sterilbereiche S, S' und S" gebildet ist (Fig. 3). Denkbar ist dabei auch, daß diese Sterilbereiche in einem in Zuführrichtung F einen Leertakt vorgebenden Abstand voneinander angeordnet sind (nicht dargestellt).

Die Gesamtansichten der Maschine 1 gemäß Fig. 1 und 2 verdeutlichen, daß in die jeweiligen Sterilgehäuse 14, 15, 16 ein insgesamt mit 17 bezeichnetes Belüftungssystem einmündet, das mit nachfolgend in ihren Steuerungs- und Verteilfunktionen näher beschriebenen Düsen in den Sterilbereichen S, S' und S" zusammenwirkt. Dieses den sterilen Schließvorgang gewährleistende, jeweilige Verteilungen von Zu- und Abluft regelnde Belüftungssystem 17 ist so ausgelegt, daß insbesondere nach einer automatischen Reinigung sämtlicher Baugruppen der Maschine 1 mit geringem Aufwand auch deren Sterilisation durchführbar ist. Dazu sind zusätzliche Reinigungsleitungen 17' (Fig. 1) vorgesehen, die nicht näher beschrieben werden.

Die vergrößerten Ausschnittsdarstellungen gemäß Fig. 6 und Fig. 7 zeigen, daß die Sterilisationsvorrichtung 7 im Nahbereich der vertikal in der Transportbahn 5 (Zuführebene B') zugeführten Kappen 3 eine ein Sterilisationsmittel anbringende Aerosol-Spülbaugruppe 19 aufweist, die mit mehreren Sprühdüsen 20 versehen ist. Die Kappen 3 werden mittels der Vorrichtung 4 so in die Transportbahn 5 eingeführt, daß die Kappen 3 im oberen Bereich der Bahn 5 mit im wesentlichen horizontal zugänglichem Innenraum 18 (Fig. 9) zur Sprühdüse 20 hinweisen.

Dieser Baugruppe 19 ist eine die Kappen 3 mit Heiß- und/oder Kaltluft spülende Trocknungsbaugruppe 21 mit mehreren Trocknungsdüsen 24 (Fig. 6, Fig. 7) nachgeordnet. Von dieser Baugruppe 21 ausgehend ist der bogenförmig verlaufende Umlenkabschnitt 11 der Transportbahn 5 vorgesehen, die damit unterseitig aus dem Sterilgehäuse 14 bzw. dem Sterilbereich S der Sterilisationsvorrichtung 7 ausmündet und die sterilisierten Kappen 3' auf vorteilhaft kurzem Weg in den zweiten Sterilbereich S' übergibt.

Die Aerosol-Sprühbaugruppe 19 (Fig. 9) weist im Bereich der Sprühdüsen 20 jeweilige sich zur auf der Transportbahn 5 befindlichen Kappen 3 hin kegelig erweiternde Abdeckteile 22 auf. Im Bereich der Abdeckteile 22 ist der in Schließstellung (Fig. 10) dem Produkt in der Flasche 2 zugewandte Innenraum 18 der Kappen 3 direkt mit einem Aerosol (Fig. 9, Pfeil C), beispielsweise einer 33 %-igen H₂O₂-Lösung, einsprühbar. Die Konstruktion ist dabei so ausgelegt, daß zwischen den kegeligen Abdeckteilen 22 und der Transportbahn 5 bzw. diese tragenden Seitenwänden 23 ein minimaler Spalt D gebildet ist und damit die mit einem Überdruck im Innenraum 18 erfolgende Spülung der Kappen 3 mit höherem Wirkungsgrad möglich ist. Die Seitenansichten gemäß Fig. 6 und 7 zeigen dabei, daß im Bereich der Spülbaugruppe 19 mehrere parallel übereinander angeordnete Sprühdüsen 20 vorgesehen sind. Unter Wirkung des Sprühdruckes C ist die Kappe zu den Führungsteilen 5" der Bahn 5 hin verlagerbar, so daß ein das H₂O₂-Aerosol ausleitender Spalt D' gebildet wird.

Ausgehend von den insbesondere drei Aerosol-Sprühdüsen 20 sind in Vorschubrichtung E der Kappen 3 vorlaufend jeweilige Trocknungsdüsen 24 der Trocknungsbaugruppe 21 vorgesehen. Für den gleichmäßig getakteten Vorschub der Kappen 3 weist diese Baugruppe 21 ebenfalls drei Trocknungsdüsen 24 auf, wobei in der dargestellten Ausführung zwischen den Baugruppen 19 und 21 zwei Leertakte vorgesehen sind (Pfeil L, Fig. 7), so daß damit ein für das H₂O₂-Aerosol eine Einwirkzeit vorgebender Transportweg definiert ist, der durch die Anzahl der Leertakte variabel vorgegeben werden kann.

In der Prinzipdarstellung gemäß Fig. 7 ist die Sterilisationsvorrichtung 7 mit dem Sterilgehäuse 14 näher dargestellt, wobei deutlich wird, daß die aus den Baugruppen 19 und 21 bzw. dem gesamten Sterilbereich S austretende Abluft 25 mit dem H₂O₂-Aerosol der Spülbaugruppe 19 mischbar ist und dieses Aerosol-Luft-Gemisch (Strich-Darstellung, Fig. 7) entgegen der Zuführrichtung E der Kappen 3 in einer Strömungsrichtung G, G' durch einen Durchlaßbereich 26 aus dem Sterilgehäuse 14 zur oberseitigen Vereinzelungsvorrichtung 4 hin ableitbar ist. Damit werden die Kappen 3 weitgehend vollständig umspült, so daß diese vorteilhaft einer Vorbehandlung ausgesetzt sind. Durch einen oberen Umlenkbereich 5' (Fig. 6) im Nahbereich des Durchlasses 26 werden die Kappen 3 so ausgerichtet, daß die Strömung G auch den Innenraum 18 erfaßt.

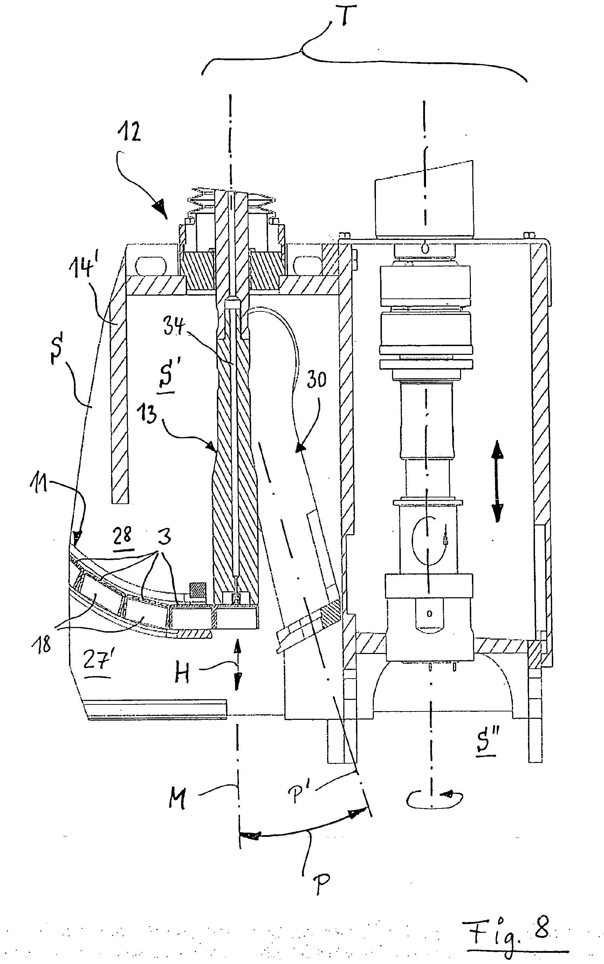

In Fig. 6 und 7 wird deutlich, daß die Sterilisationsvorrichtung 7 im Nahbereich des unteren Umlenkabschnitts 11 der Transportbahn 5 eine sich bis in den zweiten Sterilbereich S' der Aufsetzvorrichtung 12 erstreckende und im wesentlichen horizontal über der Flaschenzuführung 9 verlaufende Trennwand 27 aufweist. Oberhalb dieser Trennwand 27 mündet der Umlenkabschnitt 11 durch eine als Luftdurchlaß wirksame Öffnung 28 (Fig. 8) in der Gehäusewand 14' in den zweiten Sterilbereich S' ein. Die Größe dieser Öffnung 28 ist durch ein variabel anpaßbares Schließblech 29 (Fig. 6) in ihrem Öffnungsquerschnitt unterschiedlich ausführbar. In Fig. 10 ist dieser Übergabebereich sichtbar, wobei die untere Trennwand 27 als Abschirmung zum Bereich der nach oben offenen Flaschen 2 (Förderrichtung F) deutlich wird. Das vordere Ende der Trennwand 27 ist an einer das Ende der Transportbahn 5 erfassenden Abstützung 25 gehalten, die ihrerseits einen Durchlaß zwischen Sterilbereich S" und Trennwandkammer 27' definiert. In Fig. 6 ist mit den Strömungspfeilen Q die laminare Verteilung verdeutlicht, wobei im Bereich einer Vorderwand 14" die aus der vorgelagerten Füllzone X einlaufenden gefüllten Flaschen 2 sofort durch einen sterilen Luftstrahl vor Rekontamination geschützt werden.

Die vergrößerten Ausschnittsdarstellungen gemäß Fig. 8 und 10 bis 16 zeigen eine Ausführungsform der im zweiten Sterilbereich S" vorgesehenen Aufsetzvorrichtung 12, die mit der Schraubvorrichtung 8 die Einheit T bildet. Das vertikal verlagerbare Aufsetzorgan 13 (Bewegungspfeil H, Fig. 8) der Vorrichtung 12 wirkt in unterschiedlichen Phasen mit einem die aus dem Umlenkabschnitt 11 zugeführten Kappen 3 erfassenden Pendelhalter 30 so zusammen, daß die Kappen 3 in einer ersten Bewegungsphase erfaßt und gleichzeitig vereinzelt werden.

Der Pendelhalter 30 ist dabei zwischen dem eine vertikale Längsmittelebene M aufweisenden Aufsetzorgan 13 und einer auslaßseitigen zweiten Vertikalwand 31 des den zweiten Sterilbereich S' definierenden Gehäuseteils 15 verlagerbar, wobei eine Schwenkbewegung (Pfeil P) in bzw. entgegen der Flaschenförderrichtung F so ausführbar ist, daß die Pendelachse P' aus ihrer Deckungslage mit der vertikalen Hochachse M der Vorrichtung 12 wegschwenkbar (Fig. 10) und zu dieser rückführbar ist (Fig. 11).

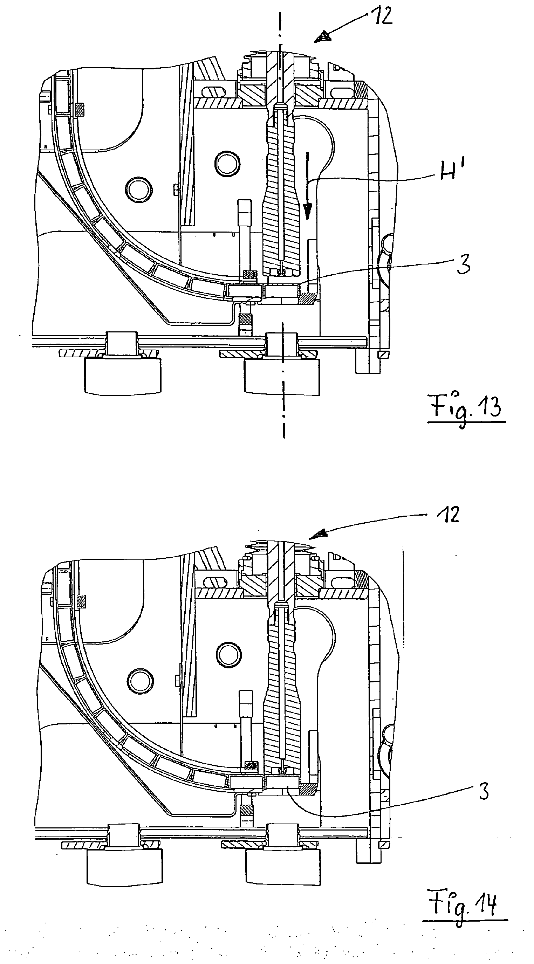

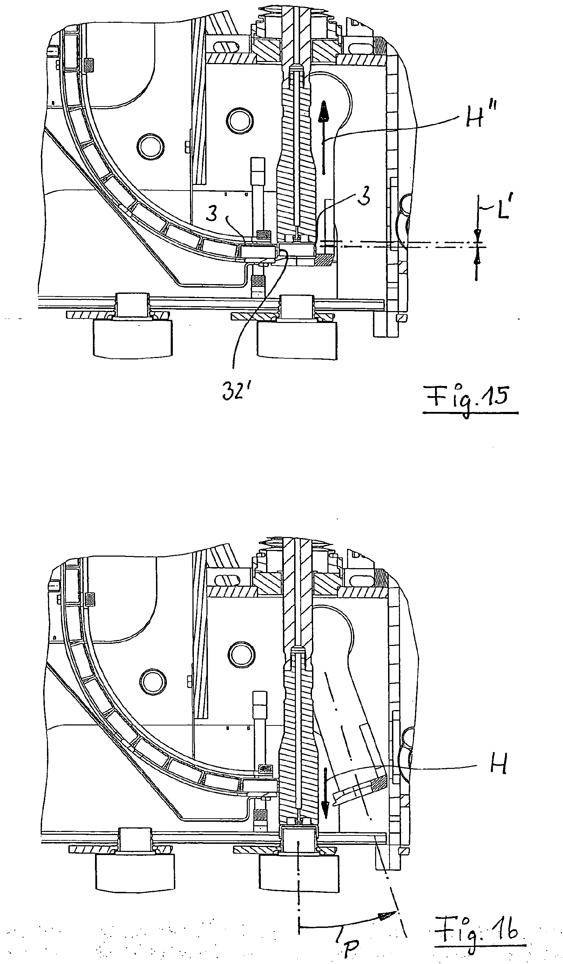

Das Aufsetzorgan 13 weist einen vorderen Zylinderteil 32 auf, der mit seiner Außenseite 33 vertikal vor der Mündung des die aufgereihten Kappen 3 zuführenden Umlenkabschnittes 11 verlagerbar ist (Fig. 10). Ausgehend von dieser im Abschnitt 11 befindlichen Reihe der sterilisierten Kappen 3 ist die jeweils vorderste der Kappen 3 einzeln dann in einer bogenförmigen Vorschubrichtung E' nachführbar, wenn der Pendelhalter 30 eine in Fig. 12 ersichtliche obere Freigabestellung aufweist. Ausgehend von dieser Phase gemäß Fig. 13 ist die Aufsetzvorrichtung 12 prinzipiell so steuerbar, daß die nachgeführte Kappe 3 vom Aufsetzorgan 13 erfaßbar ist, bei nachfolgender vertikaler Absenkbewegung (Pfeil H, Fig. 8) des Aufsetzorgans 13 die Kappe 3 auf die Flasche 2 aufgesetzt wird und dabei gleichzeitig die nächstfolgende Kappe 3 im Bereich des Umlenkabschnittes 11 mittels der Außenseite 33 des Zylinderteils 32 zurückgehalten werden kann (Fig. 16).

Dieses Handhabungssystem weist dazu ein Aufsetzorgan 13 auf, das im Bereich des Zylinderteils 32 mit einer mit Saugluft beaufschlagbaren Bohrung 34 versehen ist, die stirnseitig im Bereich einer Ringfläche 35 aus dem Zylinderteil 32 ausmündet. Denkbar ist auch, an Stelle des zylindrischen Teils 32 eine konische Gestaltung dieses Teils 32 vorzusehen (nicht dargestellt). Gemäß Fig. 13 bis 15 ist die AnsaugPhase bei Aufnahme der in den Pendelhalter 30 zugeführten Kappe 3 gezeigt, wobei in dieser Phase das Aufsetzorgan 13 um einen Hubweg H' abgesenkt (Fig. 13) und die Kappe 3 in einer Saugstellung gemäß Fig. 14 erfaßt wird. Bis zu diesem Zeitpunkt ist die Kappe 3 ausgehend von der Vorschubphase E' gemäß Fig. 12 in einem L-förmigen Aufnahmearm 36 des Pendelhalters 30 gehalten (Fig. 14). Der Aufnahmearm 36 weist dazu einen kurzen Schenkel 37 mit einer Aufnahmekontur 37' auf. Nach dem Absetzen des Aufsetzorgans 13 auf der Kappe 3 (Fig. 14) wird die Kappe 3 um einen kurzen Hubweg L' (Hubbewegung H", Fig. 15) angehoben und dabei gleichzeitig die im Umlenkabschnitt 11 der Bahn 5 aus dem Sterilbereich S nachfolgende Kappe 3 mittels des Zylinderteils 32' der angehobenen Kappe 3 zurückgehalten. In dieser Hubstellung H" gemäß Fig. 15 wird der L-förmige Aufnahmearm 36 des Pendelhalters 30 in Pfeilrichtung P vom Bereich des Aufsetzorgans 13 weggeschwenkt, so daß in Richtung der vertikalen Hochachse M die Öffnung der Flasche 2 erreichbar wird und nun die angesaugt gehaltene Kappe 3 vertikal nach unten (Pfeil H, Fig. 16) zur Flasche 2 hin verlagert werden kann.

Entsprechend einer im System wählbaren Schließlage der Kappe 3 wird dabei die Flasche 2 bereits hier dicht verschlossen (nicht dargestellt), wobei entsprechend der Kappen-Flaschen-Einheit das Aufsetzorgan 13 in Richtung zur Flasche 2 hin auf eine entsprechende Länge der vertikalen Hubbewegung H einstellbar ist. Bei der Maschine 1 wird die Kappe 3 nur in eine gemäß Fig. 17 ersichtliche Auflagestellung aufgedrückt und in einer nachfolgend näher beschriebenen Phase fest und fluiddicht verschlossen.

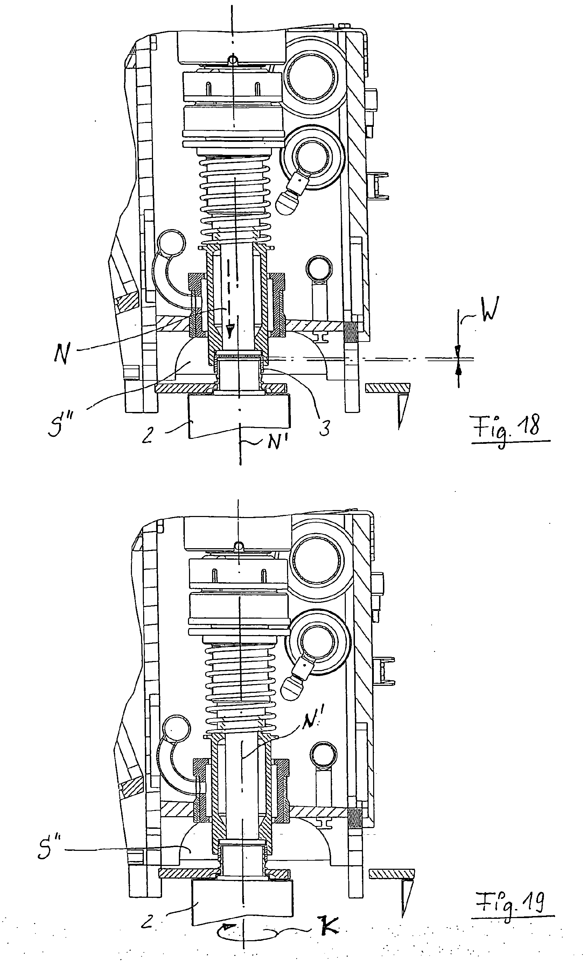

In Fig. 17 bis 19 ist die im dritten Sterilbereich S" des getakteten Linearläufers 1 vorgesehene Schließvorrichtung 8 näher veranschaulicht, wobei diese als eine Schraubbaugruppe ausgebildet ist. Die Schraubbaugruppe 38 weist dabei einen Schraubkopf 39 auf, der innerhalb einer mit Sterilluft beaufschlagbaren und dem Sterilbereich S" oberseitig vorgelagerten Schraubkammer 40 drehbar ist. Mit diesem Schraubkopf 39 wird die unter sterilen Bedingungen auf der Flasche 2 mit einem Abstand W (Fig. 17) positionierte Kappe 3 nach einer entsprechenden Absenkbewegung (Pfeil N) erfaßt (Fig. 18) und durch eine nachfolgende Dreh- bzw. Schraubbewegung (Pfeil K, Fig. 19) um die vertikale Achse N' so verlagert, daß der dichte Verschluß von Kappe 3 und Flasche 2 durch eine Schraubverbindung erreicht ist.

Der in der Schraubkammer 40 wirksame Schraubkopf 39 ist dabei mit einem oberen Dreh- und Hubantrieb 41 verbunden, der außerhalb des Gehäuses 16 angeordnet ist (Fig. 3). Damit kann - im Unterschied zum Sterilluft aufnehmenden und für die vorbeschriebene Schraubphase vorgesehenen dritten Sterilbereich S" - in der Schraubkammer 40 eine nur uitracleane Bedingungen erfordernde Luftzufuhr vorgesehen werden, so daß damit eine wesentliche Senkung des technischen Aufwandes erreicht ist. Die beiden Kammern S" und 40 sind dabei im Bereich eines mit Sterilluft beaufschlagbaren Bewegungsspaltes 42 verbunden.

Die vergrößerte Darstellung gemäß Fig. 17 zeigt, daß im Bereich des den Bereich S" und die Kammer 40 verbindenden Bewegungsspaltes 42 eine an eine Sterilluftversorgung 43 angeschlossene Ringkammer 44 vorgesehen ist und aus dieser die sterile Luft sowohl in die Schraubkammer 40 als auch in den dritten Sterilbereich S" verteilbar ist (Fig. 17, Pfeil R, R'). Es versteht sich, daß über die Sterilluftversorgung 43 auch eine Zuführung von H₂O₂-Aerosol möglich ist, wobei diese Zuführungen taktweise oder in vorgesehenen Intervallen durchführbar sind. Mit Strömungspfeilen R" ist die Wirkung mit einer in der Schraubkammer 40 vorgesehenen Absaugung deutlich. Der im Stillstand auf die Kappen 3 aufsetzbare Schraubkopf 39 ist in zweckmäßiger Ausführung mit einem Servomotor 41 als Antrieb versehen (Fig. 3).

Im Sterilbereich S" ist nahe dem in Förderrichtung F hinteren Wandteil 16" eine weitere Sterilluft-Zuführung 46 vorgesehen, die eine Schutzwirkung zum Umgebungsluft aufweisenden Bereich X' hin erzeugt. Zusätzlich zu der laminaren vertikalen Strömung U' wird durch einen Auslaßspalt 47 eine Strömung U erzeugt, so daß eine Rekontamination des Sterilbereichs S" sicher vermieden wird.

Mit diesen in Bezug auf die in der Linearläufer-Maschine 1 in Reihe angeordneten und jeweilige Sterilbereiche bildenden Sterilgehäusen 14, 15, 16 ist eine in Förderrichtung F endseitige Anordnung der bewegungsintensive Baugruppen aufweisenden Schließvorrichtung 8 bzw. der Schraubbaugruppe 38 erreicht. In dieser linearen Anordnung ist es möglich, die jeweiligen Kammern durch vertikale Strömungen mit geringem Aufwand an steriler Luft keimfrei zu halten. Bei dieser damit optimalen Steuerung der Sterilbedingungen in S, S' und S" bzw. über den gesamten Verschlußbereich T sind die jeweiligen Vertikalwände 14", 25, 31, 45 und 16" zwischen den Sterilbereichen nach Art von Schleusen wirksam, so daß die Flaschen 2 mit hoher Produktivität keimfrei verschlossen werden.

In einer weiteren, nicht näher dargestellten Ausbildung der Maschine 1 ist vorgesehen, daß vorzugsweise an Stelle der modularen Schließvorrichtung 8 mit dem Schraubkopf 39 eine in den Verfahrensablauf integrierbare Schweiß- oder Klebevorrichtung vorgesehen ist. Damit ist insbesondere ein thermischer Verschließprozeß so durchführbar, daß entsprechend ausgebildete Kappen 3 bzw. Flaschen 2 stoffschlüssig verbunden sind.

## Patentansprüche

1. Verfahren zum Verschließen von Flaschen (2) mit sterilen Kappen (3), bei dem entlang einer Transportbahn (5) einer Übergabevorrichtung zugeführte Kappen (3) sterilisiert und diese danach mit nach unten weisendem Innenraum (18) auf Flaschen (2) aufgesetzt werden, **dadurch gekennzeichnet, daß** die Kappen (3) in unsteriler Umgebung in einer vertikalen Reihung aneinandergelegt, mit in horizontaler Richtung offenem Innenraum (18) in im wesentlichen vertikaler Vorschubrichtung (Ebene B, B') einem ersten Sterilbereich (S) zugeführt, in diesem sterilisiert, in einen zweiten Sterilbereich (S') überführt, hier auf in diesen linear (Ebene A) zugeführte Flaschen (2) zur Bildung jeweiliger Flaschen-Kappen-Einheiten aufgesetzt und danach verschlossen werden.

2. Verfahren nach Anspruch 1, **dadurch gekennzeichnet, daß** die Flaschen-Kappen-Einheit entweder sofort im zweiten Sterilbereich (S') mit einer fortgesetzten vertikalen Relativbewegung (H) dicht verschlossen oder aus dem zweiten Sterilbereich (S') in einen dritten Sterilbereich (S") verlagert und hier mit den bereits aufgesetzten Kappen (3) ein dichter Flaschenverschluß hergestellt wird.

3. Verfahren nach Anspruch 1 oder 2, **dadurch gekennzeichnet, daß** der Flaschenverschluß durch eine einen Schub-Rast-Vorgang oder einen Schraubvorgang aufweisende Relativbewegung zwischen Kappe (3) und Flasche (2) hergestellt wird.

4. Verfahren nach einem der Ansprüche 1 bis 3, **dadurch gekennzeichnet, daß** die außerhalb des ersten Sterilbereichs (S) mit horizontal ausgerichtetem Innenraum (18) in jeweilige vertikale Bereiche von Transportbahnen (5) einsortierten Verschlußkappen (3) in den vertikal zu durchlaufenden ersten Sterilbereich (S) getaktet weitergeleitet, hier in einer horizontalen Sprührichtung (C) mit einem Sterilisationsmittel sterilisiert, auf einer eine Einwirkzeit vorgebenden Förderstrecke (L) zu einem Sprüh- und Trocknungsbereich geleitet, danach auf der Transportbahn (5) in eine Stellung mit nach unten weisendem Innenraum (18) umgelenkt, in dieser Stellung in den zweiten Sterilbereich (S') verlagert und hier auf die Flaschen (2) aufgesetzt werden.

5. Verfahren nach einem der Ansprüche 1 bis 4, **dadurch gekennzeichnet, daß** während der vertikalen Zuführphase der Kappen (3) eine Vorentkeimung der Kappen (3) durch ein aus dem ersten Sterilbereich (S) abströmendes Luft-Sterilisationsmittel-Gemisch (25) durchgeführt wird.

6. Verfahren nach einem der Ansprüche 1 bis 5, **dadurch gekennzeichnet, daß** die Kappen (3) beim Durchlaufen des ersten Sterilbereiches (S) zumindest phasenweise mit einem Wasserstoffperoxid (H₂O₂) enthaltenden Sterilisationsmittel besprüht werden, dieses mit Überdruck in den Innenraum (18) der Kappen (3) eingebracht wird und danach eine Trocknung zumindest durch Ausblasen des Innenraums (18) der Kappen (3) durchgeführt wird.

7. Verfahren nach einem der Ansprüche 1 bis 6, **dadurch gekennzeichnet, daß** im ersten Sterilbereich (S) gleichzeitig mehrere in der Transportbahn (5) vertikal verlagerbare Kappen (3) unter Überdruck sterilisiert und getrocknet werden.

8. Verfahren nach einem der Ansprüche 1 bis 7, **dadurch gekennzeichnet, daß** der vertikale erste Sterilbereich (S), der den Aufsetzbereich bildende zweite Sterilbereich (S') und der insbesondere zur Verschraubung der Kappen (3) vorgesehene dritte Sterilbereich (S") durch jeweilige einzeln erzeugte und sterile Luft und/oder Sterilisationsmittel enthaltende laminare Verdrängungsströmungen (Q, Q', Q") vor Rekontaminationen geschützt werden.

9. Verfahren nach Anspruch 8, **dadurch gekennzeichnet, daß** in einer Zone zwischen erstem und zweitem Sterilbereich (S, S') jeweilige eine Rekontamination sowohl der Kappen (3) als auch der gefüllten Flaschen (2) verhindernde und einen aseptischen Verschluß garantierende Verdrängungsströmungen (Q', Q") mittels steriler Luft eingeleitet werden.

10. Verfahren nach Anspruch 8 oder 9, **dadurch gekennzeichnet, daß** im Aufsetzbereich Sterilluft (Pfeil Z) zugeführt wird und diese laminar nach unten über die einlaufenden Flaschen (2) abgeleitet wird.

11. Verfahren nach einem der Ansprüche 1 bis 10, **dadurch gekennzeichnet, daß** die Kunststoff-Flaschen (2) und die Kappen (3) mehrbahnig, insbesondere 8- oder 10-bahnig, jeweils getaktet in den zweiten Sterilbereich (S') zugeführt und aus diesem als die Flaschen-Kappen-Einheit weitergeleitet werden.

12. Maschine zum sterilen Verschliessen von Flaschen (2) mit Kappen (3), womit die Kappen (3) ausgehend von einer eine Transportbahn (5) aufweisenden Vereinzelungsvorrichtung (4) der Maschine aufnehmbar, im Bereich zumindest einer Sterilkammer (6) der Maschine sterilisierbar, unter sterilen Bedingungen auf horizontal zugeführte Flaschen (2) aufsetzbar und diese mittels einer Schliessvorrichtung (8) der Maschine verschliessbar sind, **dadurch gekennzeichnet, dass** eine eine oberseitige Vereinzelungsvorrichtung (4) für die Kappen (3) aufweisende sowie diese über eine vertikale Transportbahn (5) aufnehmende Sterilisationsvorrichtung (7) der Maschine, eine von dieser ausgangsseitig die Kappen (3) übernehmende sowie mit einer horizontalen Flaschenzuführung (9) verbundene Aufsetzvorrichtung (12) der Maschine und eine zumindest ein Schliessorgan (10) aufweisende Schliessvorrichtung (8) der Maschine als getaktet zusammenwirkende Baugruppen eines Linearläufers (1) ausgebildet sind.

13. Maschine nach Anspruch 12, **dadurch gekennzeichnet, daß** die die Kappen (3) durch eine Sterilkammer (6) der Sterilisationsvorrichtung (7) vertikal hindurchleitende Transportbahn (5) endseitig einen Umlenkabschnitt (11) aufweist und dieser im Nahbereich der im zweiten Sterilbereich (S') angeordneten Aufsetzvorrichtung (12) ausmündet.

14. Maschine nach Anspruch 12 oder 13, **dadurch gekennzeichnet, daß** die Aufsetzvorrichtung (12) ein wahlweise zu betätigendes Aufsetz- und/oder Schließorgan (13) aufweist.

15. Maschine nach einem der Ansprüche 12 bis 14, **dadurch gekennzeichnet, daß** dem zweiten Sterilbereich (S') ein die Schließvorrichtung (8) aufweisender dritter Sterilbereich (S") unmittelbar nachgeordnet ist.

16. Maschine nach einem der Ansprüche 12 bis 15, **dadurch gekennzeichnet, daß** die Sterilisationsvorrichtung (7), die Aufsetzvorrichtung (12) und die Schließvorrichtung (8) mit jeweiligen Sterilgehäusen (14, 15, 16) versehen und diese oberhalb eines die Flaschen (2) horizontal zuführenden Flaschenförderteils (9) in Reihe hintereinander (Ebene A) angeordnet sind.

17. Maschine nach einem der Ansprüche 12 bis 16, **dadurch gekennzeichnet, daß** in den jeweiligen Sterilgehäusen (14, 15, 16) jeweilige keimarme und/oder sterile Luft bzw. Sterilisationsmittel eines Belüftungssystems (17) zuführende Düsen und/oder Absaugkanäle münden.

18. Maschine nach Anspruch 17, **dadurch gekennzeichnet, daß** über Reinigungsleitungen (17') nach einer automatischen Reinigung im Bereich der wesentlichen Baugruppen des Linearläufers (1) eine Sterilisation durchführbar ist.

19. Maschine nach einem der Ansprüche 12 bis 18, **dadurch gekennzeichnet, daß** die Steritisationsvorrichtung (7) im Nahbereich der vertikal in der Transportbahn (5) zugeführten Kappen (3) eine zu deren horizontal zugänglichem Innenraum (18) gerichtete Aerosol-Sprühbaugruppe (19) mit mehreren Sprühdüsen (20) aufweist, dieser Baugruppe eine die Kappen (3) mit Heiß- und/oder Kaltluft spülende Trocknungsbaugruppe (21) nachgeordnet ist und von dieser ausgehend der bogenförmig verlaufende Umlenkabschnitt (11) der Transportbahn (5) unterseitig aus dem Sterilgehäuse (14) der Sterilisationsvorrichtung (7) ausmündet.

20. Maschine nach Anspruch 19, **dadurch gekennzeichnet, daß** die Aerosol-Sprühbaugruppe (19) im Bereich der Sprühdüsen (20) jeweilige sich zu auf der Transportbahn (5) befindlichen Kappen (3) hin erweiternde Abdeckteile (22) aufweist, in deren Bereich der in Schließstellung dem Produkt zugeordnete Innenraum (18) der Kappen (3) direkt mit 32 %igem H₂O₂-Aerosol einsprühbar ist, derart, daß im Innenraum (18) der Kappen (3) ein Überdruck entsteht.

21. Maschine nach Anspruch 19 oder 20, **dadurch gekennzeichnet, daß** die Aerosol-Spülbaugruppe (19) mehrere parallel übereinander angeordnete Sprühdüsen (20) aufweist.

22. Maschine nach einem der Ansprüche 19 bis 21, **dadurch gekennzeichnet, daß** ausgehend von den drei Aerosol-Sprühdüsen (20) in Vorschubrichtung (E) der Kappen (3) mehrere Leertakte (L) vorgesehen sind und die Trocknungsbaugruppe (21) ihrerseits drei Trocknungsdüsen (24) aufweist.

23. Maschine nach einem der Ansprüche 19 bis 22, **dadurch gekennzeichnet, daß** die aus der Trocknungsbaugruppe (21) austretende Abluft mit dem H₂O₂-Aerosol der Spülbaugruppe (19) mischbar und dieses Gemisch (25) entgegen der Zuführrichtung (E) der Kappen (3) in Richtung (G, G') der oberseitigen Vereinzelungsvorrichtung (4) in einem Durchlaßbereich (26) der Transportbahn (5) aus dem Sterilgehäuse (14) ableitbar ist.

24. Maschine nach einem der Ansprüche 19 bis 23, **dadurch gekennzeichnet, daß** die Sterilisationsvorrichtung (7) im Nahbereich des unteren Umlenkabschnitts (11) der Transportbahn (5) eine sich bis in den zweiten Sterilbereich (S') der Aufsetzvorrichtung (12) erstreckende und im wesentlichen horizontal über der Flaschenzuführung (9) verlaufende Trennwand (27) aufweist.

25. Maschine nach einem der Ansprüche 19 bis 24, **dadurch gekennzeichnet, daß** der Umlenkabschnitt (11) der Transportbahn (5) oberhalb der unteren Trennwand (27) durch eine durch Strömungen (Q', Z) der Sterilluft als Schleuse wirksame Öffnung in den zweiten Sterilbereich (S') über eine erste Vertikalwand (14') einmündet.

26. Maschine nach einem der Ansprüche 12 bis 18, **dadurch gekennzeichnet, daß** die Aufsetzvorrichtung (12) im Bereich des vertikal verlagerbaren Aufsetzorgans (13) mit einem die zugeführten Kappen (3) einzeln erfassenden Pendelhalter (30) zusammenwirkt.

27. Maschine nach Anspruch 26, **dadurch gekennzeichnet, daß** der Pendelhalter (30) zwischen Aufsetzorgan (13) und einer auslaßseitigen zweiten Vertikalwand (31) des zweiten Sterilbereichs (S') in bzw. entgegen der Flaschenförderrichtung (F) schwenkbar ist.

28. Maschine nach Anspruch 26 oder 27, **dadurch gekennzeichnet, daß** das Aufsetzorgan (13) einen vorderen Zylinderteil (32) aufweist, der mit seiner Außenseite (33) vertikal vor der Mündung des die aufgereihten Kappen (3) zuführenden Umlenkabschnitts (11) verlagerbar ist, derart, daß in oberer Freigabestellung des Zylinderteils (32) von dieser Kappen-Reihe die jeweils vorderste der Kappen (3) einzeln in den Pendelhalter (30) nachführbar, diese Kappe (3) vom Aufsetzorgan (13) erfaßbar, bei nachfolgender vertikaler Absenkbewegung (Pfeil H) des Aufsetzorgans (13) die in diesem erfaßte Kappe (3) auf die Flasche (2) aufsetzbar und dabei die nächstfolgende Kappe (3) im Unlenkabschnitt (11) mittels des Zylinderteils (32) rückhaltbar ist.

29. Maschine nach einem der Ansprüche 26 bis 28, **dadurch gekennzeichnet, daß** das Aufsetzorgan (13) im Zylinderteil (32) eine mit Saugluft beaufschlagbare Bohrung (34) aufweist, die stirnseitig im Bereich einer Ringfläche (35) aus dem Zylinderteil (32) ausmündet, so daß bei auf der Oberseite der Kappe (3) aufliegender Saugstellung die Kappe (3) durch Unterdruck erfaßbar ist.

30. Maschine nach einem der Ansprüche 26 bis 29, **dadurch gekennzeichnet, daß** der Pendelhalter (30) mit einem L-förmigen Aufnahmearm (36) versehen ist, auf dessen unterhalb des Aufsetzorgans (13) positionierbarem kurzem Schenkel (37) die Kappen (3) einzeln aufschiebbar sind, derart, daß mittels der Saugleitung (34) des Aufsetzorgans (13) die Kappe (3) vom kurzen Schenkel (37) abhebbar, dabei die im Umlenkabschnitt (11) nachfolgende Kappe (3) mittels des Zylinderteils (32') der angehobenen Kappen (3) rückhaltbar, danach der L-förmige Aufnahmearm (36) vom Bereich des Aufsetzorgans (13) wegschwenkbar und nun die angesaugt gehaltene Kappe (3) vertikal nach unten zur Flasche (2) hin verlagerbar ist.

31. Maschine nach einem der Ansprüche 26 bis 30, **dadurch gekennzeichnet, daß** das Aufsetzorgan (13) der Aufsetzvorrichtung (12) in Richtung zur Flasche (2) hin auf unterschiedliche vertikale Hubwege (H) einstellbar ist.

32. Maschine nach einem der Ansprüche 26 bis 31, **dadurch gekennzeichnet, daß** das Aufsetzorgan (13) mit der Kappe (3) nach dem vertikalen Aufsetzen auf die Flasche (2) in eine eine Rastverbindung o. dgl. formschlüssigen Verbund bewirkende Schließstellung zur Flasche (2) hin linear vorschiebbar ist.

33. Maschine nach einem der Ansprüche 12 bis 18, **dadurch gekennzeichnet, daß** die Schließvorrichtung (8) des getakteten Linearläufers (1) eine Schraubbaugruppe (38) aufweist, deren Schraubkopf (39) in dem mit Sterilluft beaufschlagbaren dritten Sterilbereich (S") verlagerbar ist, derart, daß die ruhenden Kappen (3) erfaßbar und auf die Flasche (2) aufdrehbar sind.

34. Maschine nach Anspruch 33, **dadurch gekennzeichnet, daß** der Schraubkopf (39) der Schraubbaugruppe (38) über eine Schraubkammer (40) mit einem oberen Dreh- und Hubantrieb (41) verbunden und dieser außerhalb der für ultracleane Bedingungen geeigneten Schraubkammer (40) angeordnet ist.

35. Maschine nach Anspruch 33 oder 34, **dadurch gekennzeichnet, daß** der untere Sterilbereich (S") und die obere Schraubkammer (40) im Bereich eines mit Sterilluft beaufschlagbaren Bewegungsspaltes (42) verbunden sind.

36. Maschine nach einem der Ansprüche 33 bis 35, **dadurch gekennzeichnet, daß** im Bereich des die beiden Kammern (S"; 40) verbindenden Bewegungsspaltes (42) eine an eine Sterilluftversorgung (43) angeschlossene Ringkammer (44) vorgesehen und aus dieser die sterile Luft sowohl in den Sterilbereich (S") als auch in die Schraubkammer (40) verteilbar (Pfeil R, R') ist.

37. Maschine nach einem der Ansprüche 33 bis 36, **dadurch gekennzeichnet, daß** im dritten Sterilbereich (S") enthaltene Luft absaugbar (Pfeil R") und dabei eine zweite Vertikalwand (31) zwischen diesen beiden Sterilbereichen (S', S") nach Art einer Schleuse wirksam ist.

38. Maschine nach einem der Ansprüche 33 bis 37, **dadurch gekennzeichnet, daß** der im Stillstand auf die Kappen (3) aufsetzbare Schraubkopf (39) mit einem Servomotor als Antrieb (41) versehen ist.

39. Maschine nach einem der Ansprüche 12 bis 18, **dadurch gekennzeichnet, daß** diese im Bereich der Aufsetz- und/oder Schließeinheit (T) mit zumindest einer die Kappen (3) stoffschlüssig mit der Flasche (2) verbindenden Schweiß- oder Klebevorrichtung versehen ist.

40. Maschine nach Anspruch 39, **dadurch gekennzeichnet, daß** die Schweißvorrichtung an Stelle der Schließvorrichtung (8) in den Verfahrensablauf integriert ist.

## Claims

1. A method for closing bottles (2) with sterile caps (3), in the case of which caps (3), which are supplied along a transport track (5) to a transfer device, are sterilised and are then placed onto bottles (2) with the interior (18) pointing downwards, **characterised in that** the caps (3), placed side by side in a non-sterile environment in a vertical arrangement, are supplied to a first sterile area (S) with the interior (18) being open in the horizontal direction in substantially vertical feed direction (plane B, B'), are sterilized in said first sterile area (S), are transferred into a second sterile area (S'), are placed here onto bottles (2), which are supplied in a linear manner (plane A) here, to form respective bottle-cap units and are subsequently closed.

2. The method according to claim 1, **characterised in that** the bottle-cap unit is either tightly closed immediately in the second sterile area (S') with a continued vertical relatively movement (H) or is moved out of the second sterile area (S') into a third sterile area (S") and a tight bottle closure is produced here with the caps (3), which have already been placed.

3. The method according to claim 1 or 2, **characterised in that** the bottle closure is produced by means of a relative movement between cap (3) and bottle (2), which has a push-snap-on process or a screwing process.

4. The method according to any one of claims 1 to 3, **characterised in that** the closure caps (3), which have been sorted into respective vertical areas of transport tracks (5) outside of the first sterile area (S) with horizontally oriented interior (18), are transferred in a synchronized manner into the first sterile area (S), which is to be passed vertically, are sterilized here in a horizontal spraying direction (C) with a sterilisation agent, are guided on a conveying route (L), which determines an exposure time, to a spraying and drying area, are subsequently deflected on the transport track (5) into a position in which the interior (18) faces downwards, are moved into the second sterile area (S') in this position and are placed onto the bottles (2) here.

5. The method according to any one of claims 1 to 4, **characterised in that** a presterilising of the caps (3) is carried out during the vertical supply phase of the caps (3) by means of an air-sterilisation agent mixture (25), which flows from the first sterile area (S).

6. The method according to any one of claims 1 to 5, **characterised in that**, when passing through the first sterile area (S), the caps (3) are sprayed at least phase-wise with a sterilisation agent containing a hydrogen peroxide (H₂O₂), said sterilisation agent is introduced with overpressure into the interior (18) of the caps (3) and a drying is subsequently carried out at least by blowing out the interior (18) of the caps (3).

7. The method according to any one of claims 1 to 6, **characterised in that** a plurality of the caps (3), which can be moved vertically in the transport track (5), are simultaneously sterilised and dried under overpressure in the first sterile area (S),

8. The method according to any one of claims 1 to 7, **characterised in that** the vertical first sterile area (S), the second sterile area (S') forming the placement area, and the third sterile area (S"), which is in particular provided for screwing the caps (3), are protected against recontamination by respective laminar displacement flows (Q, Q', Q"), which are individually generated and which contain sterile air and/or sterilisation agents.

9. The method according to claim 8, **characterised in that** respective displacement flows (Q', Q"), which prevent a recontamination of the caps (3) as well as of the filled bottles (2) and which guarantee an aseptic closure, are introduced by means of sterile air in a zone between first and second sterile area (S, S').

10. The method according to claim 8 or 9, **characterised in that** sterile air (arrow Z) is supplied in the placement area and that said sterile air is discharged downwards in a laminar manner via the incoming bottles (2).

11. The method according to any one of claims 1 to 10, **characterised in that** the plastic bottles (2) and the caps (3) are supplied into the second sterile area (S') and are transferred from the latter as the bottle-cap unit on a plurality of tracks, in particular on 8 or 10 tracks, in each case in a synchronised manner.

12. A machine for the sterile closing of bottles (2) with caps (3), whereby, starting at a separating device (4) of the machine, which has a transport track (5), the caps (3) can be picked up, can be sterilised in the area of at least one sterile chamber (6) of the machine, can be placed onto horizontally guided bottles (2) under sterile conditions, and said bottles can be closed by means of a closing device (8) of the machine, **characterised in that** a sterilisation device (7) of the machine, which has a separating device (4) for the caps (3) on the upper side and which accommodates said caps via a vertical transport track (5), a placing device (12) of the machine, which receives the caps (3) from said sterilisation device on the exit side and which is connected to a horizontal bottle supply (9), and a closing device (8) of the machine, which has at least one closing member (10), are configured as assembly of a linear armature (1), which interact in a synchronised manner.

13. The machine according to claim 12, **characterised in that** the transport track (5), which vertically guides the caps (3) through a sterile chamber (6) of the sterilisation device (7), has a deflection section (11) on the end side and the latter exits in the immediate vicinity of the placing device (12) arranged in the second sterile area (S').

14. The machine according to claim 12 or 13, **characterised in that** the placing device (12) has a placing and/or closing member (13), which can be operated optionally.

15. The machine according to any one of claims 12 to 14, **characterised in that** a third sterile area (S"), which has the closing device (8), is arranged immediately downstream from the second sterile area (S').

16. The machine according to any one of claims 12 to 15, **characterised in that** the sterilisation device (7), the placing device (12) and the closing device (8) are provided with respective sterile housings (14, 15, 16) and the latter are arranged one behind the other in a row (plane A) above a bottle conveying part (9), which horizontally supplies the bottles (2).

17. The machine according to any one of claims 12 to 16, **characterised in that** respective nozzles and/or suction channels, which supply low-germ and/or sterile air or sterilisation agents, respectively, of a ventilation system (17), end in the respective sterile housings (14, 15, 16).

18. The machine according to claim 17, **characterised in that** a sterilisation can be carried out via cleaning lines (17') after an automatic cleaning in the area of the essential assemblies of the linear armature (1).

19. The machine according to any one of claims 12 to 18, **characterised in that**, in the immediate vicinity of the caps (3), which are vertically supplied in the transport track (5), the sterilisation device (7) has an aerosol spraying assembly (19), which is directed to the horizontally accessible interior (18) of said caps, comprising a plurality of spray nozzles (20), a drying assembly (21), which flushes the caps (3) with hot and/or cold air, is arranged downstream from this assembly, and starting therefrom, the deflection section (11) of the transport track (5), which runs in a curved manner, exits from the sterile housing (14) of the sterilisation device (7) on the bottom side.

20. The machine according to claim 19, **characterised in that**, in the area of the spray nozzles (20), the aerosol spraying assembly (19) has respective cover parts (22), which widen towards the caps (3) located on the transport track (5), in the area of which the interior (18) of the caps (3), which is assigned to the product in the closed position, can be sprayed directly with 32% H₂O₂ aerosol in such a way that an overpressure is created in the interior (18) of the caps (3).

21. The machine according to claim 19 or 20, **characterised in that** the aerosol flushing assembly (19) has a plurality of spray nozzles (20) arranged parallel on top of one another.

22. The machine according to any one of claims 19 to 21, **characterised in that**, starting at the three aerosol spray nozzles (20), a plurality of idle cycles (L) is provided in feed direction (E) of the caps (3) and the drying assembly (21), in turn, has three drying nozzles (24).

23. The machine according to any one of claims 19 to 22, **characterised in that** the exhaust air exiting from the drying assembly (21) can be mixed with the H₂O₂ aerosol of the flushing assembly (19) and this mixture (25) can be discharged from the sterile housing (14) opposite the supply direction (E) of the caps (3) in the direction (G, G') of the separating device (4) on the upper side in a passage area (26) of the transport track (5).

24. The machine according to any one of claims 19 to 23, **characterised in that**, in the immediate vicinity of the lower deflection section (11) of the transport track (5), the sterilisation device (7) has a partition wall (27), which extends into the second sterile area (S') of the placing device (12) and which runs substantially horizontally above the bottle supply (9).

25. The machine according to any one of claims 19 to 24, **characterised in that** the deflection section (11) of the transport track (5) above the lower partition wall (27) opens through an opening in the second sterile area (S'), which acts as airlock due to flows (Q', Z) of the sterile air, into the second sterile area (S') over a first vertical wall (14').

26. The machine according to any one of claims 12 to 18, **characterised in that**, in the area of the vertically movable placing member (13), the placing device (12) interacts with a pendulum holder (30), which individually seizes the supplied caps (3).

27. The machine according to claim 26, **characterised in that** the pendulum holder (30) can be pivoted between placing member (13) and a second vertical wall (31) of the second sterile area (S') on the outlet side in or opposite to the bottle conveying direction (F), respectively.

28. The machine according to claim 26 or 27, **characterised in that** the placing member (13) has a front cylinder part (32), which can be moved with its outer side (33) vertically in front of the outlet of the deflection section (11), which supplies the caps (3), which are placed in a row, in such a manner that, in upper release position of the cylinder part (32), the foremost of the caps (3) in this cap row, can individually be transferred into the pendulum holder (30), this cap (3) can be seized by the placing member (13), the cap (3) seized by the placing member (13) can be placed onto the bottle (2) in response to the subsequent vertical lowering movement (arrow H) thereof, and the next cap (3) can thereby be retained in the deflection section (11) by means of the cylinder part (32).

29. The machine according to an one of claims 26 to 28, **characterised in that** the placing member (13) in the cylinder part (32) has a bore (34), to which vacuum air can be applied and which exits frontally from the cylinder part (32) in the area of an annular surface (35), so that, the cap (3) can be seized by low pressure, in the case of the suction position bearing on the upper side of the cap (3).

30. The machine according to any one of claims 26 to 29, **characterised in that** the pendulum holder (30) is provided with an L-shaped receiving arm (36), onto the short leg (37) of which, which can be positioned below the placing member (13), the caps (3) can be pushed individually in such a way that the cap (3) can be lifted off the short leg (37) by means of the suction line (34) of the placing member (13), the cap (3) following in the deflection section (11) can thereby be retained by means of the cylinder part (32') of the lifted caps (3), the L-shaped receiving arm (36) can subsequently be pivoted away from the area of the placing member (13), and the cap (3), which is held by suction, can now be moved vertically downwards towards the bottle (2).

31. The machine according to any one of claims 26 to 30, **characterised in that** the placing member (13) of the placing device (12) can be adjusted to different vertical stroke paths (H) in the direction towards the bottle (2).

32. The machine according to any one of claims 26 to 31, **characterised in that**, after the vertical placing of the cap (3) onto the bottle (2), the placing member (13) can be linearly moved towards the bottle (2) into a closed position, which effects a snap-on connection or a similar positive bond.

33. The machine according to any one of claims 12 to 18, **characterised in that** the closing device (8) of the synchronised linear aperture (1) has a screw assembly (38), the screw head (39) of which can be moved in the third sterile area (S"), to which sterile air can be applied, in such a way that the resting caps (3) can be seized and screwed onto the bottle (2).

34. The machine according to claim 33, **characterised in that** the screw head (39) of the screw assembly (38) is connected via a screw chamber (40) to an upper rotary and lifting drive (41) and that the latter is arranged outside of the screw chamber (40), which is suitable for ultra-clean conditions.

35. The machine according to claim 33 or 34, **characterised in that** the lower sterile area (S") and the upper screw chamber (40) are connected in the area of a movement gap (42), to which sterile air can be applied.

36. The machine according to any one of claims 33 to 35, **characterised in that**, in the area of the movement gap (42), which connects the two chambers (S"; 40), an annular chamber (44) is provided, which is connected to a sterile air supply (43), and from which the sterile air can be distributed (arrow R, R') in the sterile area (S") as well as into the screw chamber (40).

37. The machine according to any one of claims 33 to 36, **characterised in that** air contained in the third sterile area (S") can be extracted by means of suction (arrow R") and a second vertical wall (31) is thereby effective between these two sterile areas (S', S") in the manner of an airlock.

38. The machine according to any one of claims 33 to 37, **characterised in that** the screw head (39), which can be placed onto the cap (3) at standstill, is provided with a servo motor as drive (41).

39. The machine according to any one of claims 12 to 18, **characterised in that**, in the area of the placing and/or closing unit (T), said machine is provided with at least one fusing or gluing device, which connects the caps (3) to the bottle (2) by means of a substance-to-substance bond.

40. The machine according to claim 39, **characterised in that** the fusing device is integrated into the process flow in place of the closing device (8).

## Revendications

1. Procédé d'obturation de bouteilles (2) avec des capuchons stériles (3), dans le cadre duquel des capuchons (3) amenés le long d'une voie de transport (5) d'un dispositif de transfert sont stérilisés puis, leur espace intérieur (18) orienté vers le bas, placés sur des bouteilles (2), **caractérisé en ce que** les capuchons (3), placés l'un contre l'autre en rangée verticale dans un milieu non stérile, leur espace intérieur (18) ouvert en sens horizontal, sont amenés dans une première zone stérile (S) selon une direction de déplacement essentiellement verticale (plan B, B'), y sont stérilisés puis transférés dans une seconde zone stérile (S') où ils sont placés sur des bouteilles (2) amenées linéairement (plan A), pour former des unités bouteille-capuchon, après quoi ils sont fermés.

2. Procédé selon la revendication 1, **caractérisé en ce que** l'unité bouteille-capuchon est soit immédiatement obturée de façon étanche dans la seconde zone stérile (S') par un mouvement relatif vertical continu (H), soit transférée de la seconde zone stérile (S') dans une troisième zone stérile (S") où une obturation étanche des bouteilles est réalisée avec les capuchons (3) déjà placées sur celles-ci.

3. Procédé selon la revendication 1 ou 2, **caractérisé en ce que** l'obturation des bouteilles est réalisée par un mouvement relatif entre le capuchon (3) et la bouteille (2), lequel mouvement comporte un processus de poussée et d'enclenchement ou un processus de vissage.

4. Procédé selon l'une des revendications 1 à 3, **caractérisé en ce que** les capuchons de fermeture (3) agencés avec leur espace intérieur (18) orienté horizontalement dans les sections verticales respectives de voies de transport (5) à l'extérieur de la première zone stérile (S) sont transférés à une certaine cadence dans la première zone stérile (S) qu'ils parcourent verticalement, y sont stérilisés avec agent de stérilisation pulvérisé en direction horizontale (C) puis, le long d'une distance de transport (L) dont la longueur détermine un temps d'action, sont transférés vers une zone d'aspersion et de séchage puis, sur la voie de transport (5), sont tournés en une position telle que leur espace intérieur (18) soit orienté vers le bas et, dans cette position, sont amenés dans la seconde zone stérile (S') où ils sont placés sur les bouteilles (2).

5. Procédé selon l'une des revendications 1 à 4, **caractérisé en ce que,** pendant la phase d'avance verticale des capuchons (3), une pré-désinfection des capuchons (3) est effectuée au moyen d'un mélange (25) d'air et d'agent de stérilisation s'échappant de la première zone stérile (S).

6. Procédé selon l'une des revendications 1 à 5, **caractérisé en ce que** les capuchons (3), lorsqu'ils parcourent la première zone stérile (S), sont, au moins par phases, aspergés d'un agent de stérilisation contenant de l'eau oxygénée (H₂O₂), cet agent étant introduit avec une surpression dans l'espace intérieur (18) des capuchons (3), après quoi est effectué un séchage au moins par soufflage de l'espace intérieur (18) des capuchons (3).

7. Procédé selon l'une des revendications 1 à 6, **caractérisé en ce que** plusieurs capuchons (3) déplaçables verticalement dans la voie de transport (5) sont stérilisé sous pression et séchés à la fois dans la première zone stérile (S).

8. Procédé selon l'une des revendications 1 à 7, **caractérisé en ce que** la première zone stérile (S) verticale, la seconde zone stérile (S') qui forme la zone de mise en place des capuchons et la troisième zone stérile (S") notamment prévue pour le vissage des capuchons (3) sont respectivement protégées contre les recontaminations par des flux de refoulement laminaires (Q, Q', Q") produits individuellement et contenant de l'air stérile et/ou des agent de stérilisation.

9. Procédé selon la revendication 8, **caractérisé en ce que** des flux de refoulement (Q', Q") empêchant la recontamination aussi bien des capuchons (3) que des bouteilles remplies (2) et garantissant une obturation aseptique sont introduits au moyen d'air stérile dans une zone entre la première et la seconde zone stérile (S, S').

10. Procédé selon la revendication 8 ou 9, **caractérisé en ce que** de l'air stérile (flèche Z) est introduit dans la zone de mise en place des capuchons puis dirigé en un flux laminaire vers le bas au-dessus des bouteilles (2) entrant dans la zone.

11. Procédé selon l'une des revendications 1 à 10, **caractérisé en ce que** les bouteilles en matière plastique (2) et les capuchons (3) sont respectivement amenés à une certaine cadence sur plusieurs voies, notamment sur 8 ou 10 voies, dans la seconde zone stérile (S') d'où ils ressortent pour être transférés sous forme d'unités bouteille-capuchon.

12. Machine pour l'obturation stérile de bouteilles (2) par des capuchons (3) avec laquelle les capuchons (3) peuvent, à partir d'un dispositif séparateur (4) de la machine, lequel dispositif présente une voie de transport (5), être repris puis stérilisés dans la zone d'au moins une chambre stérile (6) de la machine puis, dans des conditions stériles, placés sur des bouteilles (2) amenées horizontalement et dont la fermeture est assurée au moyen d'un dispositif d'obturation (8) de la machine **caractérisée en ce qu'**un dispositif de stérilisation (7) de la machine muni d'un dispositif séparateur supérieur (4) pour les capuchons (3) et recevant ces capuchons par une voie de transport verticale (5), un dispositif (12) de mise en place des capuchons prenant en charge les capuchons (3) à la sortie du dispositif de stérilisation (7) et relié à un système horizontal (9) d'introduction de bouteilles et un dispositif d'obturation (8) présentant au moins un organe d'obturation (10) sont réalisés sous forme de modules d'une machine linéaire (1) agissant conjointement de façon cadencée.

13. Machine selon la revendication 12, **caractérisée en ce que** la voie de transport (5) qui transporte les capuchons (3) verticalement à travers une chambre stérile (6) du dispositif de stérilisation (7) présente à son extrémité une section de déviation (11) aboutissant à proximité du dispositif (12) de mise en place des capuchons agencé dans la seconde zone stérile (S').

14. Machine selon la revendication 12 ou 13, **caractérisée en ce que** le dispositif de mise en place des capuchons (12) présente un organe de pose et/ou d'obturation (13) pouvant être actionné au choix.

15. Machine selon l'une des revendications 12 à 14, **caractérisée en ce qu'**une troisième zone stérile (S") présentant le dispositif d'obturation (8) est agencée immédiatement en aval de la seconde zone stérile (S').

16. Machine selon l'une des revendications 12 à 15, **caractérisée en ce que** le dispositif de stérilisation (7), le dispositif de mise en place des capuchons (12) et le dispositif d'obturation (8) sont respectivement munis de carters stériles (14, 15, 16) agencés en rangée l'un derrière l'autre (plan A) au-dessus du système horizontal (9) d'introduction des bouteilles (2).

17. Machine selon l'une des revendications 12 à 16, **caractérisée en ce que** dans les carters stériles respectifs (14, 15, 16) débouchent des buses et/ou canaux d'aspiration pour l'adduction d'air pauvre en germes et/ou stérile ou d'un agent de stérilisation d'un système d'aération (17).

18. Machine selon la revendication 17, **caractérisée en ce que** des conduites de nettoyage (17') permettent, après un nettoyage automatique dans la zone des principaux modules de la machine linéaire (1), d'effectuer une stérilisation.

19. Machine selon l'une des revendications 12 à 18, **caractérisée en ce que** le dispositif de stérilisation (7) présente à proximité des capuchons (3) amenés verticalement dans la voie de transport (5) un module de pulvérisation d'aérosol (19) avec plusieurs buses de pulvérisation (20) orienté vers l'espace intérieur (18) des capuchons accessible horizontalement, et **en ce qu'**en aval de ce module est agencé un module de séchage (21) balayant les capuchons (3) avec de l'air chaud et/ou froid et que, partant de ce dernier, la section de déviation (11) arquée de la voie de transport (5) débouche sous le carter stérile (14) du dispositif de stérilisation (7).

20. Machine selon la revendication 19, **caractérisée en ce que** le module (19) de pulvérisation d'aérosol présente au niveau des buses de pulvérisation (20) des pièces de recouvrement (22) s'évasant respectivement en direction des capuchons (3) se trouvant dans la voie de transport (5), pièces au niveau desquelles un aérosol d'H₂O₂ à 32 % peut être directement pulvérisé dans l'espace intérieur (18) des capuchons (3) correspondant au produit en position de fermeture, et ce de façon à obtenir une surpression dans l'espace intérieur (18) des capuchons (3).

21. Machine selon la revendication 19 ou 20, **caractérisée en ce que** le module de pulvérisation d'aérosol (19) présente plusieurs buses de pulvérisation (20) agencées parallèlement l'une au-dessus de l'autre.

22. Machine selon l'une des revendications 19 à 21, **caractérisée en ce qu'**en aval des trois buses de pulvérisation d'aérosol (20), plusieurs cycles à vide (L) sont prévus dans le sens de l'avance (E) des capuchons (3), et **en ce que** le module de séchage (21) présente lui-même trois buses de séchage (24).

23. Machine selon l'une des revendications 19 à 22, **caractérisée en ce que** l'air évacué du module de séchage (21) peut être mélangé avec l'aérosol d'H₂O₂ du module de pulvérisation d'aérosol (19) et que ce mélange (25) peut, en sens inverse du sens d'avance (E) des capuchons (3), s'échapper du carter stérile (14) en direction (G, G') du dispositif séparateur supérieur (4) par un passage (26) de la voie de transport (5).

24. Machine selon l'une des revendications 19 à 23, **caractérisée en ce que** le dispositif de stérilisation (7) présente à proximité de la section de déviation (11) de la voie de transport (5) une cloison de séparation (27) qui s'étend essentiellement horizontalement au-dessus du système (9) d'introduction de bouteilles jusque dans la seconde zone stérile (S') du dispositif (12) de mise en place des capuchons.

25. Machine selon l'une des revendications 19 à 24, **caractérisée en ce que** la section de déviation (11) de la voie de transport (5) au-dessus de la cloison de séparation (27) inférieure débouche dans la seconde zone stérile (S') par une ouverture dans une première cloison verticale (14'), cette ouverture faisant office de sas du fait des courants (Q', Z) d'air stérile.

26. Machine selon l'une des revendications 12 à 18, **caractérisée en ce que,** dans la zone de l'organe d'obturation (13) déplaçable verticalement, le dispositif (12) de mise en place des capuchons agit conjointement avec un support oscillant (30) qui saisit un à un les capuchons (3) à leur arrivée.

27. Machine selon la revendication 26, **caractérisée en ce que** le support oscillant (30) peut pivoter dans le sens (F) de transport des bouteilles ou en sens inverse entre l'organe d'obturation (13) et une seconde cloison verticale (31) côté sortie de la seconde zone stérile (S').

28. Machine selon la revendication 26 ou 27, **caractérisée en ce que** l'organe d'obturation (13) présente une partie cylindrique avant (32) déplaçable verticalement avec sa paroi extérieure (33) devant l'embouchure de la section de déviation (11) qui amène les capuchons (3) alignés, ce déplacement se faisant de façon telle que, en position supérieure de libération de la partie cylindrique (32), le premier capuchon (3) de cette rangée de capuchons peut être introduit individuellement dans le support oscillant (30), ce capuchon (3) pouvant alors être saisi par l'organe d'obturation (13) puis, par le mouvement suivant d'abaissement vertical (flèche H) de l'organe d'obturation (13), placé sur la bouteille (2), le capuchon (3) suivant pouvant être retenu dans la section de déviation (11) au moyen de la partie cylindrique (32).

29. Machine selon l'une des revendications 26 à 28, **caractérisée en ce que** l'organe d'obturation (13) présente dans sa partie cylindrique (32) un alésage (34) pouvant servir de canal d'aspiration débouchant frontalement de la partie cylindrique (32) au niveau d'une surface annulaire (35), de sorte qu'en position d'aspiration en contact avec la face supérieure du capuchon (3) celui-ci peut être saisi par dépression.

30. Machine selon l'une des revendications 26 à 29, **caractérisée en ce que** le support oscillant (30) est muni d'un bras porte-pièce (36) en forme de L sur la branche courte (37) duquel positionnable sous l'organe d'obturation (13) les capuchons (3) peuvent placés individuellement de sorte à pouvoir être soulevés de la branche courte (37) au moyen du canal d'aspiration (34) de l'organe d'obturation (13), le capuchon (3) suivant dans la section de déviation (11) pouvant être retenu au moyen de la partie cylindrique (32') du capuchon (3) soulevé, après quoi le bras porte-pièce (36) en forme de L peut pivoter pour s'écarter de l'organe d'obturation (13) et le capuchon (3) maintenu par dépression peut être déplacé verticalement vers le bas et la bouteille (2).

31. Machine selon l'une des revendications 26 à 30, **caractérisée en ce que** différentes courses verticales (H) en direction de la bouteille (2) sont réglables pour l'organe d'obturation (13) du dispositif (12) de mise en place des capuchons.

32. Machine selon l'une des revendications 26 à 31, **caractérisée en ce que** l'organe d'obturation (13) avec le capuchon (3), après la mise en place verticale sur la bouteille (2), peut être avancé linéairement vers la bouteille (2) en une position de fermeture causant un enclenchement ou une liaison similaire à complémentarité de forme.

33. Machine selon l'une des revendications 12 à 18, **caractérisée en ce que** le dispositif d'obturation (8) de la machine linéaire (1) présente un module de vissage (38) dont la tête de vissage (39) peut être déplacée dans la troisième zone stérile (S") alimentée en air stérile de sorte que les capuchons (3) immobiles peuvent être saisis et vissés sur les bouteilles (2).

34. Machine selon la revendication 33, **caractérisée en ce que** la tête de vissage (39) du module de vissage (38) est relié par une chambre de vissage (40) à un entraînement (41) de rotation et de levage, lequel entraînement est agencé à l'extérieur de la chambre de vissage (40) conçue pour opérer dans des conditions ultrapropres.

35. Machine selon la revendication 33 ou 34, **caractérisée en ce que** la zone stérile inférieure (S") et la chambre de vissage supérieure (40) sont reliées au niveau d'un écartement (42) pouvant être alimenté en air stérile et permettant des mouvements.

36. Machine selon l'une des revendications 33 à 35, **caractérisée en ce qu'**une chambre annulaire (44) branchée sur une alimentation en air stérile (43) est prévue au niveau de l'écartement (42) reliant les deux chambres (S"; 40), l'air stérile pouvant, à partir de cette chambre (44), être distribué aussi bien dans la zone stérile (S") que dans la chambre de vissage (40) (flèche R, R') ist.

37. Machine selon l'une des revendications 33 à 36, **caractérisée en ce que** l'air contenu dans la troisième zone stérile (S") peut être aspiré (flèche R"), une seconde cloison verticale (31) faisant fonction de sas entre ces deux zone stérile (S', S").

38. Machine selon l'une des revendications 33 à 37, **caractérisée en ce que** la tête de vissage (39) pouvant être placée sur les capuchons (3) immobiles est entraînée par un servomoteur (41).

39. Machine selon l'une des revendications 12 à 18, **caractérisée en ce qu'**elle est équipée, au niveau de l'unité d'obturation ou de fermeture (T), d'au moins un dispositif de soudage ou de collage assurant une jonction entre les matériaux des capuchons (3) et des bouteilles (2).

40. Machine selon la revendication 39, **caractérisée en ce que** le dispositif de soudage est intégré dans le cycle opérationnel au lieu du dispositif d'obturation (8).
